# EUROPEAN PATENT SPECIFICATION

(11) **EP 3 325 661 B1**
(45) Date of publication and mention of the grant of the patent: **16.10.2019**
(21) Application number: 16757058.9
(22) Date of filing: 21.07.2016
(51) Int. Cl.: C12Q 1/68

(54) **FGFR EXPRESSION AND SUSCEPTIBILITY TO AN FGFR INHIBITOR**
FGFR-EXPRESSION UND ANFÄLLIGKEIT FÜR EINEN FGFR-INHIBITOR
EXPRESSION DE FGFR ET SENSIBILITÉ À UN INHIBITEUR DU FGFR

(30) Priority: 24.07.2015 WO PCT/IB2015/001245; 14.03.2016 WO PCT/IB2016/000290
(43) Date of publication of application: 30.05.2018
(73) Proprietor: Debiopharm International SA, 1006 Lausanne (CH)
(72) Inventor: VASLIN-CHESSEX, Anne, 1008 Prilly (CH); MOULON, Corinne, 1806 Saint-Légier (CH); BRICHORY, Franck, 47380 Bonne (FR); POKORSKA-BOCCI, Anna, 1135 Denens (CH)
(74) Representative: KATZAROV S.A.
(86) International application number: PCT/IB2016/001044
(87) International publication number: WO 2017/017516

(56) References cited:
- EP-A1- 2 695 950
- WO-A2-2014/179448
- Y. NAKANISHI ET AL: "The Fibroblast Growth Factor Receptor Genetic Status as a Potential Predictor of the Sensitivity to CH5183284/Debio 1347, a Novel Selective FGFR Inhibitor", MOLECULAR CANCER THERAPEUTICS, vol. 13, no. 11, 28 August 2014 (2014-08-28), pages 2547-2558, XP055258714, US ISSN: 1535-7163, DOI: 10.1158/1535-7163.MCT-14-0248
- V. GUAGNANO ET AL: "FGFR Genetic Alterations Predict for Sensitivity to NVP-BGJ398, a Selective Pan-FGFR Inhibitor", CANCER DISCOVERY, vol. 2, no. 12, 20 September 2012 (2012-09-20), pages 1118-1133, XP055141813, ISSN: 2159-8274, DOI: 10.1158/2159-8290.CD-12-0210

### SEQUENCE LISTING

<110> DEBIOPHARM INTERNATIONAL SA
<120> FGFR EXPRESSION AND SUSCEPTIBILITY TO AN FGFR INHIBITOR
<130> DPH 126 - PCT
<160> 45
<170> PatentIn version 3.5
<210> 1
   <211> 100
   <212> DNA
   <213> Homo sapiens
<400> 1
   tgtctgctga ctccagtgca tccatgaact ctggggttct tctggttcgg ccatcacggc 60
   tctcctccag tgggactccc atgctagcag gggtctctga 100
<210> 2
   <211> 100
   <212> DNA
   <213> Homo sapiens
<400> 2
   ccctgggaga gggctgcttt gggcaggtgg tgttggcaga ggctatcggg ctggacaagg 60
   acaaacccaa ccgtgtgacc aaagtggctg tgaagatgtt 100
<210> 3
   <211> 100
   <212> DNA
   <213> Homo sapiens
<400> 3
   gacgcaacag agaaagactt gtcagacctg atctcagaaa tggagatgat gaagatgatc 60
   gggaagcata agaatatcat caacctgctg ggggcctgca 100
<210> 4
   <211> 100
   <212> DNA
   <213> Homo sapiens
<400> 4
   caacccagag gagcagctct cctccaagga cctggtgtcc tgcgcctacc aggtggcccg 60
   aggcatggag tatctggcct ccaagaagtg catacaccga 100
<210> 5
   <211> 100
   <212> DNA
   <213> Homo sapiens
<400> 5
   agccaggaat gtcctggtga cagaggacaa tgtgatgaag atagcagact ttggcctcgc 60
   acgggacatt caccacatcg actactataa aaagacaacc 100
<210> 6
   <211> 100
   <212> DNA
   <213> Homo sapiens
<400> 6
   ggagacaggt aacagtttcg gctgagtcca gctcctccat gaactccaac accccgctgg 60
   tgaggataac aacacgcctc tcttcaacgg cagacacccc 100
<210> 7
   <211> 100
   <212> DNA
   <213> Homo sapiens
<400> 7
   ataagctgac actgggcaag cccctgggag aaggttgctt tgggcaagtg gtcatggcgg 60
   aagcagtggg aattgacaaa gacaagccca aggaggcggt 100
<210> 8
   <211> 100
   <212> DNA
   <213> Homo sapiens
<400> 8
   aaagatgatg ccacagagaa agacctttct gatctggtgt cagagatgga gatgatgaag 60
   atgattggga aacacaagaa tatcataaat cttcttggag 100
<210> 9
   <211> 100
   <212> DNA
   <213> Homo sapiens
<400> 9
   tatgacatta accgtgttcc tgaggagcag atgaccttca aggacttggt gtcatgcacc 60
   taccagctgg ccagaggcat ggagtacttg gcttcccaaa 100
<210> 10
   <211> 100
   <212> DNA
   <213> Homo sapiens
<400> 10
   tagcagccag aaatgttttg gtaacagaaa acaatgtgat gaaaatagca gactttggac 60
   tcgccagaga tatcaacaat atagactatt acaaaaagac 100
<210> 11
   <211> 100
   <212> DNA
   <213> Homo sapiens
<400> 11
   ctggtccttc ggggtgttaa tgtgggagat cttcacttta gggggctcgc cctacccagg 60
   gattcccgtg gaggaacttt ttaagctgct gaaggaagga 100
<210> 12
   <211> 100
   <212> DNA
   <213> Homo sapiens
<400> 12
   agtccaacgc gtccatgagc tccaacacac cactggtgcg catcgcaagg ctgtcctcag 60
   gggagggccc cacgctggcc aatgtctccg agctcgagct 100
<210> 13
   <211> 100
   <212> DNA
   <213> Homo sapiens
<400> 13
   cttcggccag gtggtcatgg cggaggccat cggcattgac aaggaccggg ccgccaagcc 60
   tgtcaccgta gccgtgaaga tgctgaaaga cgatgccact 100
<210> 14
   <211> 100
   <212> DNA
   <213> Homo sapiens
<400> 14
   gctgcccgca atgtgctggt gaccgaggac aacgtgatga agatcgcaga cttcgggctg 60
   gcccgggacg tgcacaacct cgactactac aagaagacaa 100
<210> 15
   <211> 100
   <212> DNA
   <213> Homo sapiens
<400> 15
   gatgtcctcc cgccaagcca tgttagaaaa tgcatctgac atcaagctgg agaagttcag 60
   catctccgct catggcaagg agctgttcgt caatgcagac 100
<210> 16
   <211> 100
   <212> DNA
   <213> Homo sapiens
<400> 16
   tgcagaagga gatcactgcc ctggcaccca gcacaatgaa gatcaagatc attgctcctc 60
   ctgagcgcaa gtactccgtg tggatcggcg gctccatcct 100
<210> 17
   <211> 100
   <212> DNA
   <213> Homo sapiens
<400> 17
   ggggatcggg atggagtcat gccaaaaatg gacatcattt ctggaacact tggcaaagcc 60
   tttggttgtg ttggagggta catcgccagc acgagttctc 100
<210> 18
   <211> 100
   <212> DNA
   <213> Homo sapiens
<400> 18
   cgggcattcc tgaagctgac agcattcggg ccgagatgtc tcgctccgtg gccttagctg 60
   tgctcgcgct actctctctt tctggcctgg aggctatcca 100
<210> 19
   <211> 100
   <212> DNA
   <213> Homo sapiens
<400> 19
   gggtatcaac ccagcaaaca ttggcttcag taccctgact atggagtctg acaaattcat        60
   ctgcattaga gaaaaagtag gagagcaggc ccaggtggta                              100
<210> 20
   <211> 100
   <212> DNA
   <213> Homo sapiens
<400> 20
   acaacatcgc ctgcgttatc ctcaccttca aggagccctt tggcactgag ggtcgcgggg        60
   gctatttcga tgaatttggg atcatccggg acgtgatgca                              100
<210> 21
   <211> 100
   <212> DNA
   <213> Homo sapiens
<400> 21
   cactcctcca cctttgacgc tggggctggc attgccctca acgaccactt tgtcaagctc        60
   atttcctggt atgacaacga atttggctac agcaacaggg                              100
<210> 22
   <211> 100
   <212> DNA
   <213> Homo sapiens
<400> 22
   cggtcgtgat gtggtctgtg gccaacgagc ctgcgtccca cctagaatct gctggctact        60
   acttgaagat ggtgatcgct cacaccaaat ccttggaccc                              100
<210> 23
   <211> 100
   <212> DNA
   <213> Homo sapiens
<400> 23
   tgtgatgaag gagatgggag gccatcacat tgtagccctc tgtgtgctca agggggccta        60
   taaattcttt gctgacctgc tggattacat caaagcactg                              100
<210> 24
   <211> 100
   <212> DNA
   <213> Homo sapiens
<400> 24
   aacttcctgg ctccttcact gaacatgcct agtccaacat tttttcccag tgagtcacat 60
   cctgggatcc agtgtataaa tccaatatca tgtcttgtgc 100
<210> 25
   <211> 100
   <212> DNA
   <213> Homo sapiens
<400> 25
   ggaagtattc ttcgtgttac tacagctgac ccatatgcca gtggaaaaat cagccagttt 60
   ctggggattt gcattcagag atcaggaaga ggacttggag 100
<210> 26
   <211> 100
   <212> DNA
   <213> Homo sapiens
<400> 26
   gcaagaagta tgctgaggct gtcactcggg ctaagcagat tgtgtggaat ggtcctgtgg 60
   gggtatttga atgggaagct tttgcccggg gaaccaaagc 100
<210> 27
   <211> 100
   <212> DNA
   <213> Homo sapiens
<400> 27
   ggagaactcg gccttagaat actttggtga gatgttaaag gctgctggct acaatttcta 60
   tggcaccgag aggttatata gtggcatcag tgggctagaa 100
<210> 28
   <211> 100
   <212> DNA
   <213> Homo sapiens
<400> 28
   ttccaagaag ccaaagactc cttcgcttac tgtcttcctg ttgggccagt ccgctcgaga 60
   tgctgagaga gccaaggata ttctgtgccg tctggagcat 100
<210> 29
   <211> 100
   <212> DNA
   <213> Homo sapiens
<400> 29
   tttctccatg cccaggagga ggtgaagcga atccaaagca tcccgctggt catcggacaa 60
   tttctggagg ctgtggatca gaatacagcc atcgtgggct 100
<210> 30
   <211> 100
   <212> DNA
   <213> Homo sapiens
<400> 30
   ccaatgcccg aatgccagag aaggtcacat ggatgaggag aatgaggatt ttgcgccggc 60
   tgctcagaag ataccgtgaa tctaagaaga tcgatcgcca 100
<210> 31
   <211> 100
   <212> DNA
   <213> Homo sapiens
<400> 31
   cgaaatgttt cattgtggga gcagacaatg tgggctccaa gcagatgcag cagatccgca 60
   tgtcccttcg cgggaaggct gtggtgctga tgggcaagaa 100
<210> 32
   <211> 100
   <212> DNA
   <213> Homo sapiens
<400> 32
   tggaggggca ggcttgcgag ctgcatttgg cctttctgag gcagggttta atacagcatg 60
   tgttaccaag ctgtttccta ccaggtcaca cactgttgca 100
<210> 33
   <211> 100
   <212> DNA
   <213> Homo sapiens
<400> 33
   cttctaagcc agttgtgggg attatttacc ctcctccaga ggtcagaaat attgttgaca 60
   agactgccag ctttgtggcc agaaacgggc ctgaatttga 100
<210> 34
   <211> 100
   <212> DNA
   <213> Homo sapiens
<400> 34
   acagtgaatc ttggttgtaa acttgaccta aagaccattg cacttcgtgc ccgaaacgcc 60
   gaatataatc ccaagcggtt tgctgcggta atcatgagga 100
<210> 35
   <211> 100
   <212> DNA
   <213> Homo sapiens
<400> 35
   tggtggatct agaacctggg accatggact ctgttcgctc aggtcctttt ggccagatct 60
   ttagaccaga caactttgta tttggtcagt ctggggcagg 100
<210> 36
   <211> 100
   <212> DNA
   <213> Homo sapiens
<400> 36
   ggggtaaacc cgaacaattc tgcgcgaggt agggaggcca tggcgtccgg cagtaactgg 60
   ctctccgggg tgaatgtcgt gctggtgatg gcctacggga 100
<210> 37
   <211> 100
   <212> DNA
   <213> Homo sapiens
<400> 37
   agatcccatt tcattctgaa ggccggcatc cccgttcctt aatgggcaag aatttccgct 60
   cctacctgct ggatctgcga aacactagta cgcctttcaa 100
<210> 38
   <211> 100
   <212> DNA
   <213> Homo sapiens
<400> 38
   attcgactct ccagggttca ggatatcaag tatgagcccc agctccttgc agatgatgat        60
   gctagactac tacaactgga aacccaggga aatcaaagtt                             100
<210> 39
   <211> 100
   <212> DNA
   <213> Homo sapiens
<400> 39
   ggagctggac cgcgagcgac agaaactaga gacccaggag aagaaaatca ttgcagacat        60
   taagaagatg gccaagcaag gccagatgga tgctgttcgc                             100
<210> 40
   <211> 100
   <212> DNA
   <213> Homo sapiens
<400> 40
   aagttgcccc agatccagaa gatcatgatg gagtttgagc ggcaggcaga gatcatggat        60
   atgaaggagg agatgatgaa tgatgccatt gatgatgcca                             100
<210> 41
   <211> 100
   <212> DNA
   <213> Homo sapiens
<400> 41
   tgcgggccaa catccaggct gtgtccctca agatccagac actcaagtcc aacaactcga        60
   tggcacaagc catgaagggt gtcaccaagg ccatgggcac                             100
<210> 42
   <211> 100
   <212> DNA
   <213> Homo sapiens
<400> 42
   tgctgaattc caaccatgag ttgcctgatg tttctgagtt catgacaaga ctcttctctt        60
   caaaatcatc tggcaaatct agcagcggca gcagtaaaac                             100
<210> 43
   <211> 100
   <212> DNA
   <213> Homo sapiens
<400> 43
   ggttgtcaga ctgccctatc ctctccaaat gaaatcttca ggtccacctt cttactttat        60
   taaaagggaa tcgtggggct ggacagactt tctaatgaac                              100
<210> 44
   <211> 100
   <212> DNA
   <213> Homo sapiens
<400> 44
   caccgagggt cgagccgtgc tgcacgtggc tctgcggaac cggtcaaaca cacccatcct        60
   ggtagacggc aaggatgtga tgccagaggt caacaaggtt                              100
<210> 45
   <211> 100
   <212> DNA
   <213> Homo sapiens
<400> 45
   ggcggctccg acctgggacc cctcatggtg actgaagccc ttaagccata ctcttcagga        60
   ggtccccgcg tctggtatgt ctccaacatt gatggaactc                              100

## Description

### FIELD OF THE INVENTION

The present invention relates to selection of subjects having tumors for treatment with an FGFR inhibitor as well as to the treatment of such subjects with such inhibitor.

### BACKGROUND OF THE INVENTION

Fibroblast growth factors and their receptors (FGFR) drive important developmental signaling pathways that affect cell proliferation, migration and survival. Aberrant FGF signaling plays a role in many cancers. Turner, N. and Grose, R. (2010) Nat. Rev. Cancer 10: 116-29. The FGFR family consists of FGFR1, FGFR2, FGFR3 and FGFR4. FGFR are tyrosine kinases that are activated in a fraction of tumors by gene amplification, mutations, or chromosomal translocations or rearrangements. Amplification of FGFR1 occurs in squamous cell lung carcinoma and estrogen receptor-positive breast cancer. FGFR2 is also amplified in gastric and breast cancers. Mutations in FGFR2 are observed in endometrial cancer and of FGFR3 in bladder cancer. The Cancer Genome Atlas Network (2012) Nature 489: 519-25; Elbauomy Elsheikh, S. et al. (2007) Breast Cancer Res. 9: R23; Turner, N. et al. (2010) Cancer Res. 70: 2085-94; Peng, D.F. et al. (2003) J. Pathol. 201: 439-50; Matsumoto, K. et al. (2012) Br. J. Cancer 106: 727-32; Byron, S.A. et al. (2008) Cancer Res. 68: 6902-7; Cappellen, D. et al. (1999) Nat. Genet. 23: 18-20; Al-Ahmadie, H.A. et al. (2011) J. Pathol. 224: 270-9. Chromosomal translocations and rearrangements of FGFR1, FGFR2 and FGFR3 were reported in various cancers. Parker, B.C. et al. (2014) J. Pathol. 232: 4-15.

FGFR fusion genes were also reported in various cancers, both in hematological and solid tumor types. For example, FGFR1-ERLIN2 in breast cancer, FGFR2- KIAA1967 in squamous cell lung cancer, FGFR3 translocation t(4,14) in multiple myeloma. It is noted that some fusions occur in different cancers. FGFR3-TACC3 fusions occur, for example, in glioblastoma, bladder cancer and squamous cell carcinoma. Comprehensive listings of known fusion genes are found, e.g., in Annala et al. (2013) or in Shaw, A.T. et al. (2013) Nature Reviews Cancer 13: 772-87.

Responding to a clear need, a number of more or less specific FGFR inhibitors were developed. They include PD173074 (Mohammadi et al. (1998) EMBO J. 17: 5896-904), Pazopanib (Harris et al. (2009) J. Med. Chem. 51: 4632-40; Keisner and Shah (2011) Drugs 71: 443-54), AZD4547 (Gavine et al. (2012) Cancer Res. 72: 2045-56), Ponatinib (or AP24534) (Huang et al. (2010) J. Med. Chem. 53: 4701-19), Dovitinib (Trudel et al. (2005) Blood 105: 2941-8; Man et al. (2014) J. Cell. Mol. Med. 18: 143-55), BGJ398 (Guagnano et al. (2011) J. Med. Chem. 54: 7066-83), E-3810 also known as Lucitanib (Bello et al. (2011) Cancer Res. 71: 1396-405), JNJ-42756493 (Squires et al. (2008) AACR Abstract 1545), ARQ 087 (Yu et al. (2011) Cancer Res. 71 (Suppl. 1) 3671), LY2874455 (Zhao et al. (2011) Mol Cancer Ther. 10: 2200-10), BAY1163877 (Heroult et al. (2014) Cancer Res. 74 (Suppl. 19) - Abstract 1739), ASP5878 (73rd Annual Meeting of the Japanese Cancer Association (2014) - Abstract/Poster 1411), E7090 (Saori Watanabe Miyano et al. (2015) AACR Abstract 770), ODM-203 (Holmström et al. 26th ENA Symposium (2014) Eur. J. Cancer 50(S6):142 - Abstract 432), Nintedanib (Roth et al. (2015) J Med Chem. 58: 1053-63), TAS-120 (Ochiiwa, et al. (2013) AACR; Mol. Cancer Ther. 12 (11 Suppl), Abstract A270), PRN 1109 and PRN 1371 (both in: Phan VT. et al. 26th ENA Symposium (2014) Eur. J. Cancer 50(S6):157 - Abstract 483). They also include the inhibitory aminopyrazole derivatives and their pharmaceutically acceptable salts described in international patent publication WO 2011/016528, including in particular 5-amino-1-(2-methyl-1H-benzimidazol-5-yl)-1H-pyrazol-4-yl]-(1H-indol-2-yl)-methanone, herein referred to as Compound A.

Now that several FGFR inhibitors are being administered to human subjects, the question arises as to the proper criteria for determining whether a particular subject should or should not be treated with a particular inhibitor.

Y. NAKANISHI et al.: "The Fibroblast Growth Factor Receptor Genetic Status as a Potential Predictor of the Sensitivity to CH5183284/Debio 1347, a Novel Selective FGFR Inhibitor" MOLECULAR CANCER TERAPEUTICS, vol. 13, no. 11, 28 August 2014 (2014-08-28), pages 2547-2558, XP55258714, discloses that "FGF receptors (FGFR) are tyrosine kinases that are constitutively activated in a subset of tumors by genetic alterations such as gene amplifications, point mutations, or chromosomal translocations/rearrangements. Recently, small-molecule inhibitors that can inhibit the FGFR family as well as the VEGF receptor (VEGFR) or platelet-derived growth factor receptor (PDGFR) family displayed clinical benefits in cohorts of patients with FGFR genetic alterations. However, to achieve more potent and prolonged activity in such populations, a selective FGFR inhibitor is still needed". It is reported the identification of CH5183284/Debio 1347, a selective and orally available FGFR1, FGFR2, and FGFR3 inhibitor that has a unique chemical scaffold. By interacting with unique residues in the ATP-binding site of FGFR1, FGFR2, or FGFR3, CH5183284/Debio 1347 selectively inhibits FGFR1, FGFR2, and FGFR3 but does not inhibit kinase insert domain receptor (KDR) or other kinases. Consistent with its high selectivity for FGFR enzymes. CH5183284/Debio 1347 displayed preferential antitumor activity against cancer cells with various FGFR genetic alterations in a panel of 327 cancer cell lines and in xenograft models. Because of its unique binding mode. CH5183284/Debio 1347 can inhibit FGFR2 harboring one type of the gatekeeper mutation that causes resistance to other FGFR inhibitors and block FGFR2 V564F-driven tumor growth. CH5183284/Debio 1347 is under clinical investigation for the treatment of patients harboring FGFR genetic alterations.

EP2695950 A1 (BLACKFIELD AG [DE]) relates to a method for predicting the responsiveness of a mammalian tumor cell or cancer cell to an inhibitor of a fibroblast growth factor receptor (FGFR), and to a method for predicting the responsiveness of an individual to an inhibitor of a fibroblast growth factor receptor (FGFR). These methods involve the evaluation of the status of FGFR and the status of c-myc, whereby said status of FGFR and of c-myc is indicative for the responsiveness to the inhibitor. The status of FGFR may be its amplification status or its expression level and the status of c-myc may be its expression level, like an intermediate or high expression level of c-myc. Furthermore, a kit useful for carrying out the methods described herein as well as an oligo- or polynucleotide and/or antibodies capable of detecting the expression level or gene amplification status of FGFR or the expression level of c-myc for predicting the responsiveness to the inhibitor are described.

WO2014179448 A2 (FIVE PRIME THERAPEUTICS INC [US]; GLAXOSMITHKLINE IP NO 2 LTD [GB]) discloses methods of treating cancers comprising FGFR1 gene amplification, FGFR1 overexpression, FGFR3 overexpression, FGFR3 amplification, FGF2 overexpression, and/or FGF2 gene amplification. In some embodiments, the methods comprise administering a fibroblast growth factor receptor 1 (FGFR1) extracellular domain (ECD) and/or an FGFR1 ECD fusion molecule. In some embodiments, the methods comprise administering a FGFR1 ECD and/or an FGFR1 ECD fusion molecule in combination with at least one additional therapeutic agent. In some embodiments, methods of treating cancers comprising administering a FGFR1 ECD and/or an FGFR1 ECD fusion molecule in combination with at least one chemotherapeutic agent are disclosed.

The question relates to whether an inhibitor is active against a tumor harboring a mutationally activated FGFR, a tumor expressing an FGFR fusion protein and/or a tumor containing an amplified, unmutated or mutated FGFR gene. A related question concerns the specificity for particular FGFR family members. Answers to these questions may be inhibitor-specific. Present practice appears to be to treat any tumor containing any of the above-described genetic alterations in an FGFR gene with the preferred FGFR inhibitor. Clearly, proper selection criteria are not yet known.

### SUMMARY OF THE INVENTION

The present invention relates to a method for identifying and selecting a group of subjects diagnosed with a cancer that are likely to respond to a therapeutic regimen that comprises administration of a pharmaceutical composition comprising an effective amount of Compound A, consisting of 5-amino-1-(2-methyl-1H-benzimidazol-5-yl)-1H-pyrazol-4-yl]-(1H-indol-2-yl)-methanone. The method comprises determining the levels of expression of FGFR1, FGFR2 and FGFR3 in a tumor or liquid biopsy from a subject suffering from a cancer, and if the determined level of expression of at least one of FGFR1, FGFR2 and FGFR3 exceeds a pre-established threshold level for that FGFR, determining that the subject has a cancer likely to respond to an effective amount of Compound A.

In the above-described method, levels of expression of FGFR1, FGFR2 and FGFR3 are determined at the messenger RNA (mRNA) level and the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

Messenger RNA levels may be assessed by any suitable method. Preferred is detection of FGFR mRNA molecules by digital methods. A specific method of this type is the nCounter Gene Expression Assay of NanoString used herein. Another suitable method is reverse transcription and quantitative PCR.

To establish threshold levels for each of FGFR1, FGFR2 and FGFR3, a balanced set of patient-derived tumor xenograft (PDX) models is assembled which models fit into four different categories which are similarly represented in the set:
(i) increased copy number of an FGFR gene and overexpression of an FGFR, (ii) increased copy number of an FGFR gene without overexpression of an FGFR, (iii) no increase in copy number of an FGFR gene but overexpression of an FGFR, and (iv) expression of an FGFR fusion gene product.

Levels of expression of FGFR1, FGFR2 and FGFR3 and efficacy of treatment with Compound A are measured. These data establish the relationship between response rates and percentile cutoff levels of FGFR expression. See Table 1. "Response Rate" (sometimes abbreviated as "RR") refers to the percentage of models whose tumors are effectively treated by Compound A, i.e., that meet the required minimal treatment efficacy ΔT/ΔC (here 0). A percentile cutoff level of expression of an FGFR refers to a level at which a percentile of models has the same or a lower expression level for the FGFR. For example, the 75^{th} percentile cutoff level of expression with respect to an FGFR identifies a level of the FGFR (threshold level) that corresponds to the highest level measured in the 75% of models having the lowest levels of the FGFR. It is noted that, whereas Table 1 provides threshold levels for certain percentile cutoff levels of expression, threshold levels can be easily determined for other percentile cutoff levels. The data needed for such determinations are provided in Table 2 below.

Threshold FGFR levels corresponding to cutoff levels of between about 44% and about 73% may be selected as this range of threshold levels does not exclude any model that is capable of responding to Compound A while substantially increasing the response rate observed within the models considered overexpressed, as compared to the response rate observed within all models (e.g. by more than 10%). Corresponding response rates are from about 36.2% to about 52.5%.

### More preferred pre-established threshold level of at least one FGFR corresponds to:

(a) any level corresponding to cutoff levels from about 60% to about 73%. Corresponding response rates are from about 39.6% to about 52.5%, or
(b) any levels corresponding to cutoff levels from about 65% to about 73%. Corresponding response rates are from about 42.9% to about 52.5%, or
(c) any level corresponding to cutoff levels from about 70% to about 73%.

The 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

Less preferred threshold values - because of lower response rates - are those corresponding to cutoff levels below about 44%. Also less preferred because a fraction of treatable subjects is excluded are those corresponding to cutoff levels exceeding 73%.

It is noted that for the purposes of the indication-independent method discussed here, FGFR1, 2 and 3 were considered in the calculations for each model, i.e. a model was considered overexpressed if the expression level of at least one of FGFR1, FGFR2 or FGFR3 exceeds the corresponding threshold level for the chosen percentile cutoff level.

**Table 1: Percentile cutoff, response rate and threshold FGFR values**

| Percentile Cutoff FGFR expression | Response Rate of models considered overexpressed | Response Rate of models considered not overexpressed | Threshold FGFR Levels * | | | % increase over RR observed within all models |
|---|---|---|---|---|---|---|
| | | | FGFR1 | FGFR2 | FGFR3 | |
| 40 | 34.4 | 0.0 | 0.063 | 0.212 | 0.113 | 7.6 |
| 41 | 35.0 | 0.0 | 0.077 | 0.225 | 0.116 | 9.1 |
| 42 | 35.0 | 0.0 | 0.089 | 0.238 | 0.119 | 9.1 |
| 43 | 35.0 | 0.0 | 0.097 | 0.249 | 0.123 | 9.1 |
| *44* | *36.2* | *0.0* | *0.104* | *0.257* | *0.128* | *12.1* |
| 45 | 36.2 | 0.0 | 0.109 | 0.282 | 0.134 | 12.1 |
| 50 | 36.2 | 0.0 | 0.152 | 0.389 | 0.174 | 12.1 |
| 55 | 37.5 | 0.0 | 0.240 | 0.510 | 0.210 | 15.2 |
| 60 | 39.6 | 0.0 | 0.301 | 0.669 | 0.289 | 19.7 |
| 65 | 42.9 | 0.0 | 0.484 | 0.884 | 0.490 | 25.8 |
| 70 | 48.8 | 0.0 | 0.558 | 0.984 | 0.671 | 34.8 |
| 71 | 50.0 | 0.0 | 0.577 | 1.012 | 0.703 | 36.4 |
| 72 | 50.0 | 0.0 | 0.618 | 1.045 | 0.785 | 36.4 |
| *73* | *52.5* | 0.0 | *0.641* | *1.094* | *0.815* | 39.4 |
| 74 | 52.6 | 3.6 | 0.659 | 1.153 | 0.822 | 39.5 |
| 75 | 52.6 | 3.6 | 0.698 | 1.227 | 0.831 | 39.5 |
| 80 | 57.6 | 6.1 | 0.759 | 1.460 | 0.925 | 44.7 |
| 81 | 61.3 | 5.7 | 0.840 | 1.537 | 0.950 | 48.1 |
| 82 | 63.3 | 5.6 | 0.945 | 1.588 | 0.972 | 49.8 |
| 83 | 63.3 | 5.6 | 1.078 | 1.610 | 0.991 | 49.8 |
| 84 | 62.1 | 8.1 | 1.362 | 1.854 | 1.011 | 48.7 |
| 85 | 59.3 | 12.8 | 1.645 | 2.024 | 1.035 | 46.3 |
| 90 | 71.4 | 13.3 | 2.657 | 3.975 | 1.435 | 55.5 |
| 95 | 91.7 | 18.5 | 6.489 | 23.187 | 2.712 | 65.3 |

| | | | | | | |
|---|---|---|---|---|---|---|
| * Levels of mRNA expression of FGFR exon 14 relative to the median of levels of mRNA expression of 16 reference (housekeeping) genes (ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6) determined by the nCounter Gene Expression Assay. "Response Rate" (RR) refers to percentage of models selected whose tumors are effectively treated by Compound A, i.e., that meet the required minimal treatment efficacy ΔT/ΔC (here 0). | | | | | | |

The above-described method does not discriminate between cancer indications, i.e. is indication-independent. The invention also encompasses methods for identifying and selecting a group of subjects diagnosed with a specific type of cancer, e.g., esophageal cancer, lung cancer (e.g. squamous NSCLC) or bladder cancer, that are likely to respond to a therapeutic regimen that comprises a pharmaceutical composition comprising an effective amount of Compound A. The method comprises determining the level of expression of the FGFR in a tumor or liquid biopsy from a subject suffering from the latter type of cancer (or multiple FGFR) known to be elevated in tumors of a fraction of subjects suffering from cancers of this type, and if the determined level of expression of the FGFR exceeds a pre-established threshold level, considering or declaring the subject eligible for the therapeutic regimen.

To establish a threshold level for the FGFR in question (or multiple FGFR), a balanced set of tumor xenograft (PDX) models for the specific type of cancer concerned may be assembled, whereby the models typically fit into four different categories which are similarly represented in the set:
(i) increased copy number of the FGFR gene and overexpression of the FGFR, (ii) increased copy number of the FGFR gene without overexpression of the FGFR, (iii) no increase in copy number of the FGFR gene but overexpression of an FGFR, and (iv) expression of an FGFR fusion gene product.

Levels of expression of the FGFR in question as well as efficacy of treatment with Compound A are measured. These data establish the relationship between response rates and percentile cutoff levels of FGFR expression. Threshold FGFR levels corresponding to cutoff levels or ranges of cutoff levels may be selected that are as elevated as possible without excluding any model that is capable of responding to Compound A (see the above discussion of the indication-independent method). Based on the selection objective, higher or lower percentile cutoff levels may be chosen.

The present invention also relates to a method of selecting a subject suffering from a cancer for treatment with Compound A, the method comprising determining the levels of expression of FGFR1, FGFR2 and FGFR3 in a tumor or liquid biopsy from the subject and if the determined level of expression of at least one of FGFR1, FGFR2 and FGFR3 exceeds a pre-established threshold level, considering the subject eligible for treatment, wherein levels of expression of FGFR1, FGFR2 and FGFR3 are measured at the messenger RNA level or at the protein level, and wherein the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

The present invention further relates to Compound A for use in the treatment of cancer in a subject, wherein the level of expression of at least one of FGFR1, FGFR2 and FGFR3 as determined in a tumor or liquid biopsy from the subject exceeds a pre-established threshold level
wherein the levels of expression of FGFR1, FGFR2 and FGFR3 are measured at the messenger RNA level and the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

In yet a more specific embodiment, the pre-established threshold level of at least one FGFR corresponds to any level from the 60% to the 73% cutoff level of expression for the at least one FGFR, corresponding to expression levels relative to the median of levels of mRNA expression of the above-defined set of 16 reference genes measured by the nCounter Gene Expression Assay from 0.301 to 0.641 for FGFR1, from 0.669 to 1.094 for FGFR2, and from 0.289 to 0.815 for FGFR3. In yet a more specific embodiment, the pre-established threshold level of at least one FGFR corresponds to any level from the 65% to the 73% cutoff level of expression for the at least one FGFR, corresponding to expression levels relative to the median of levels of mRNA expression of the above-defined set of 16 reference genes measured by the nCounter Gene Expression Assay from 0.484 to 0.641 for FGFR1, from 0.884 to 1.094 for FGFR2, and from 0.490 to 0.815 for FGFR3. In yet a more specific embodiment, the pre-established threshold level of at least one FGFR corresponds to any level from the 70% to the 73% cutoff level of expression for the at least one FGFR, corresponding to expression levels relative to the median of levels of mRNA expression of a set of the above-defined 16 reference genes measured by the nCounter Gene Expression Assay from 0.558 to 0.641 for FGFR1, from 0.984 to 1.094 for FGFR2, and from 0.671 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

In another specific embodiment of the present invention, the cancer is gastric cancer and the pre-established threshold level of expression of FGFR2 corresponds to
(a) any level from the 44% to the 83% cutoff level of expression for FGFR2, corresponding to expression levels relative to the median of levels of mRNA expression of the above-defined set of 16 reference genes measured by the nCounter Gene Expression Assay from 0.257 to 1.610 for FGFR2 or
(b) any levels corresponding to cutoff levels from about 60% and about 83% (0.669 - 1.610), or
(c) any levels corresponding to cutoff levels from about 70% and about 83% (0.984 - 1.610), or
(d) any levels corresponding to cutoff levels from about 80% and about 83% (1.460 - 1.610).

The 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6. In such embodiment, the level of expression of FGFR2 only, or of FGFR1, 2 and 3, may be determined.

In yet another embodiment of the present invention, the cancer is esophageal squamous-cell carcinoma and the pre-established threshold level of expression of FGFR1 corresponds to
(a) any level from the 44% to the 84% cutoff level of expression for FGFR1, corresponding to expression levels relative to the median of levels of mRNA expression of the above-defined set of 16 reference genes measured by the nCounter Gene Expression Assay from 0.104 to 1.362 for FGFR1, or
(b) any level corresponding to cutoff levels from about 60% and about 84% (0.301 - 1.362), or
(c) any level corresponding to cutoff levels from about 70% and about 84% (0.558 - 1.362), or
(d) any level corresponding to cutoff levels from about 80% and about 84% (0.759 - 1.362).

The 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

In such embodiment, the level of expression of FGFR1 only, or of FGFR1, 2 and 3, may be determined.

All the above-mentioned threshold levels, as well as threshold levels for some intermediate percentile cutoff levels, are found in Table 1. As mentioned previously, threshold levels can be easily determined for other percentile cutoff levels.

### BRIEF DESCRIPTION OF FIGURES

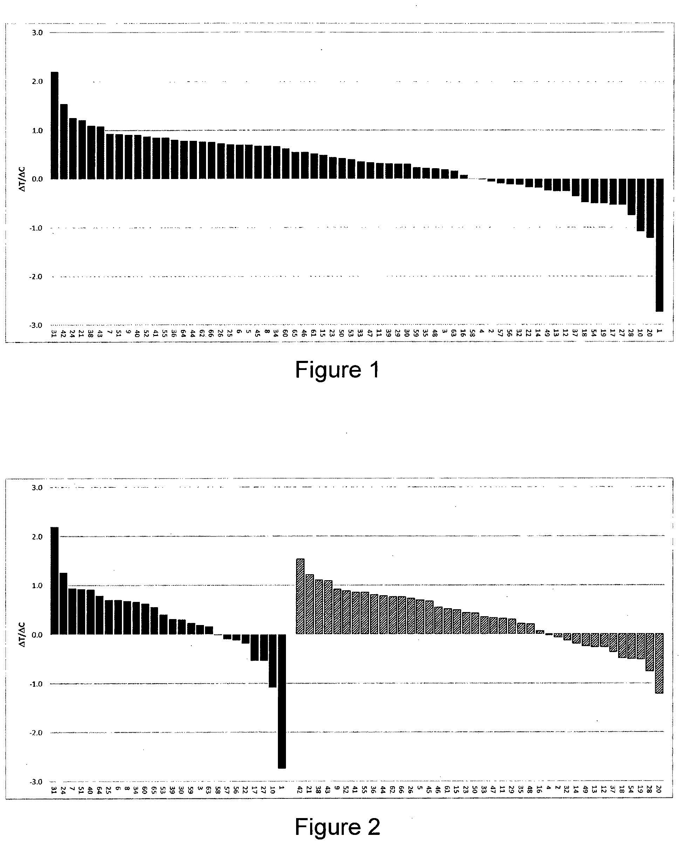

Figure 1 shows a waterfall plot of efficacy of treatment (ΔT/ΔC) with Compound A in 66 PDX models. In this analysis, a ΔT/ΔC value < 0 indicates efficacious treatment. Models are identified on the abscissa.
Figure 2. On the left is a waterfall plot of efficacy of treatment (ΔT/ΔC) with Compound A for PDX models showing neither a gain in gene copy number for any FGFR nor expression of a fusion gene. On the right is a similar plot for PDX models showing a gain in gene copy number for an FGFR or expression of a fusion gene. A ΔT/ΔC value < 0 indicates efficacious treatment. Models are identified on the abscissa.

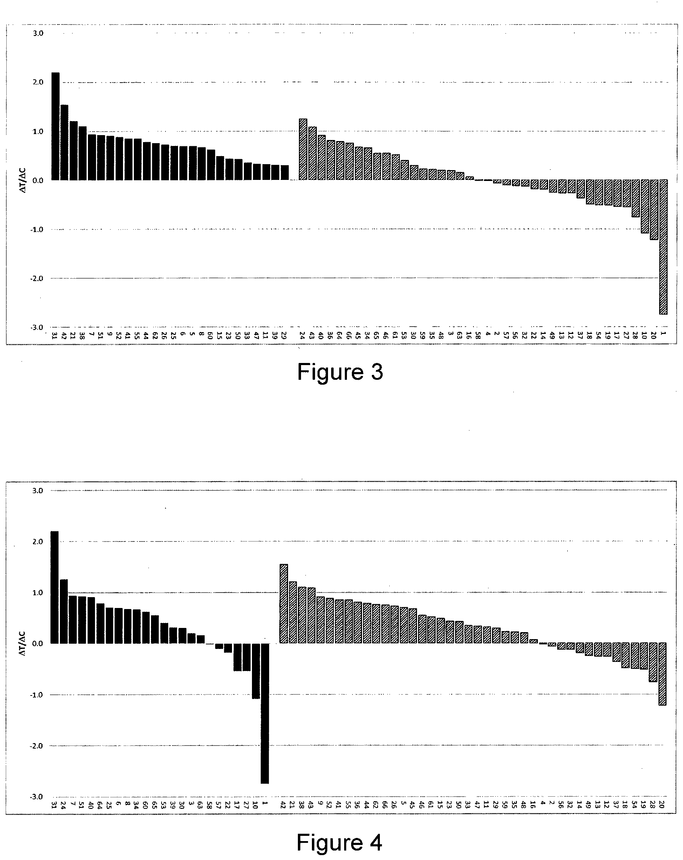

Figure 3. On the left is a waterfall plot of efficacy of treatment (ΔT/ΔC) with Compound A for PDX models not overexpressing any of FGFR1, FGFR2 or FGFR3 (levels below the 73^{th} percentile cutoff level of expression). On the right is a similar plot for PDX models overexpressing any of FGFR1, FGFR2 or FGFR3 (levels above the 73^{th} percentile cutoff level of expression). A ΔT/ΔC value < 0 indicates efficacious treatment. Models are identified on the abscissa.
Figure 4. On the left is a waterfall plot of efficacy of treatment (ΔT/ΔC) with Compound A for PDX models showing neither a gain in gene copy number for any FGFR, expression of a fusion gene or presence of an FGFR mutation. On the right is a similar plot for PDX models showing a gain in gene copy number for an FGFR or expression of a fusion gene or presence of an FGFR mutation. A ΔT/ΔC value < 0 indicates efficacious treatment. Models are identified on the abscissa.

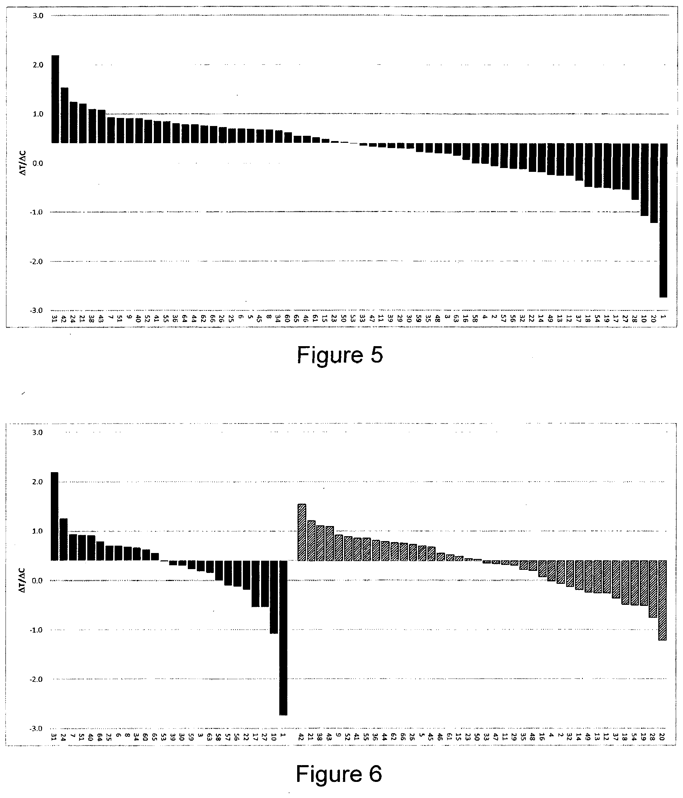

Figure 5 shows a waterfall plot of efficacy of treatment (ΔT/ΔC) with Compound A in 66 PDX models. In this analysis, a ΔT/ΔC value < 0.4 indicates efficacious treatment. Models are identified on the abscissa.
Figure 6. On the left is a waterfall plot of efficacy of treatment (ΔT/ΔC) with Compound A for PDX models showing neither a gain in gene copy number for any FGFR nor expression of a fusion gene. On the right is a similar plot for PDX models showing a gain in gene copy number for any FGFR or expression of a fusion gene. A ΔT/ΔC value < 0.4 indicates efficacious treatment. Models are identified on the abscissa.

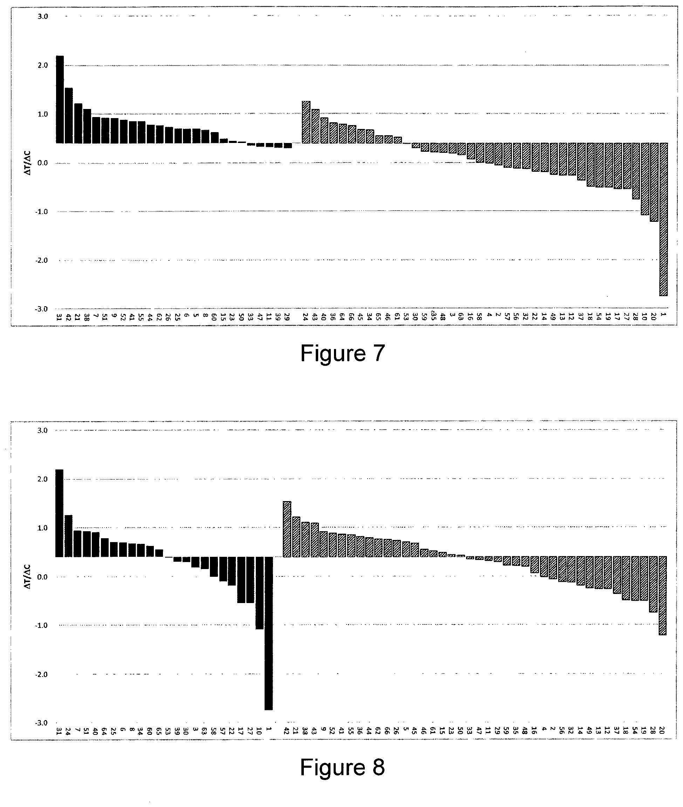

Figure 7. On the left is a waterfall plot of efficacy of treatment (ΔT/ΔC) with Compound A for PDX models not overexpressing any of FGFR1, FGFR2 or FGFR3 (levels below the 73^{th} percentile cutoff level of expression). On the right is a similar plot for PDX models overexpressing any of FGFR1, FGFR2 or FGFR3 (levels above the 73^{th} percentile cutoff level of expression). A ΔT/ΔC value < 0.4 indicates efficacious treatment. Models are identified on the abscissa.
Figure 8. On the left is a waterfall plot of efficacy of treatment (ΔT/ΔC) with Compound A for PDX models showing neither a gain in gene copy number for any FGFR, expression of a fusion gene or presence of an FGFR mutation. On the right is a similar plot for PDX models showing a gain in gene copy number for an FGFR or expression of a fusion gene or presence of an FGFR mutation. A ΔT/ΔC value < 0.4 indicates efficacious treatment. Models are identified on the abscissa.

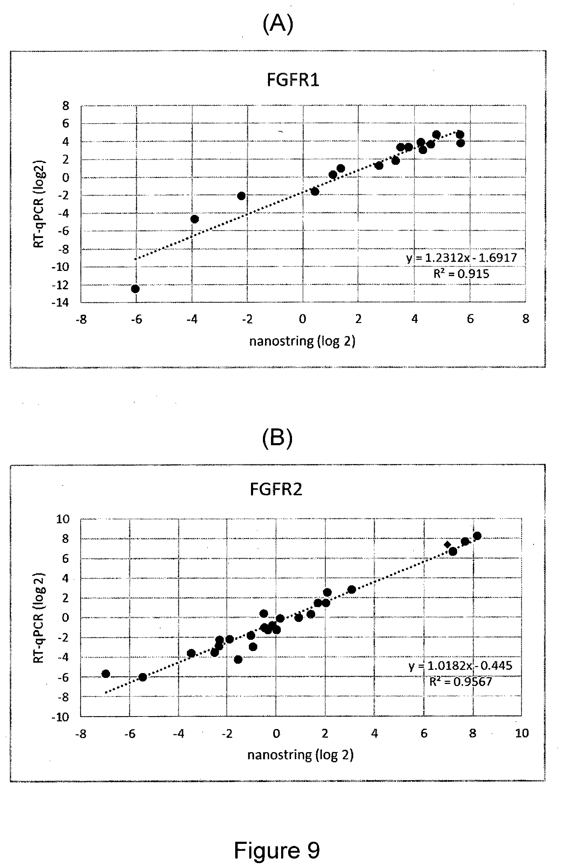

Figure 9 shows the very good correlation between mRNA levels measured by each of RT-qPCR and NanoString technologies on test sets of PDX tumor samples. A common set of three housekeeping genes (ALAS1, CLTC, MRPL19) was utilized for standardization in both methods. (A) 16 esophageal squamous-cell carcinoma (ESCC) PDX tumor samples - R² = 0.915 for FGFR1 mRNA levels; (B) 26 gastric PDX tumor samples - R² = 0.957 for FGFR2 mRNA levels.

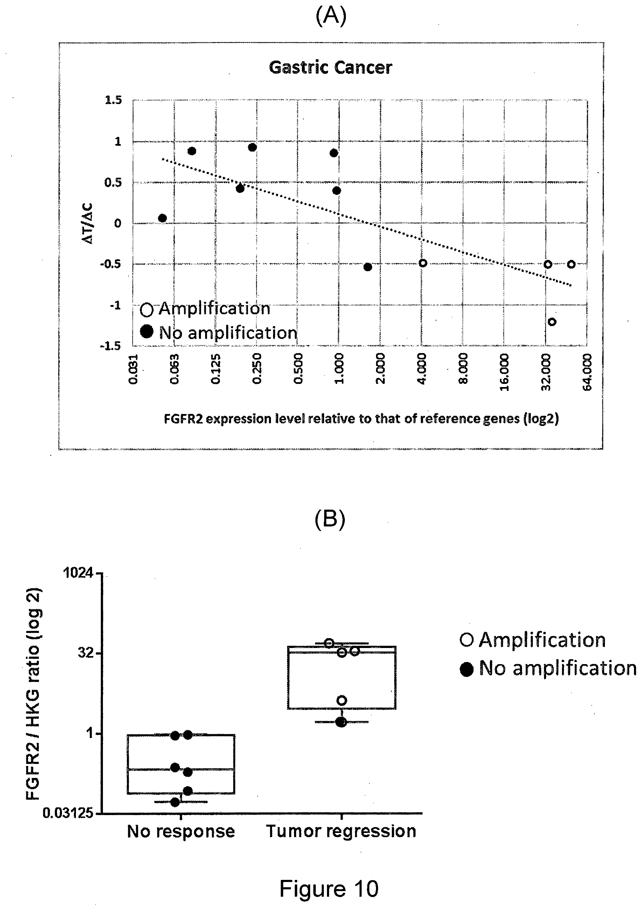

Figure 10 shows the relationship between efficacies of treatment (ΔT/ΔC) with Compound A and levels of expression of FGFR2 in PDX models of gastric cancer while indicating FGFR2 amplification (empty circles) as per Example 1. (A) correlation plot; (B) box plot.

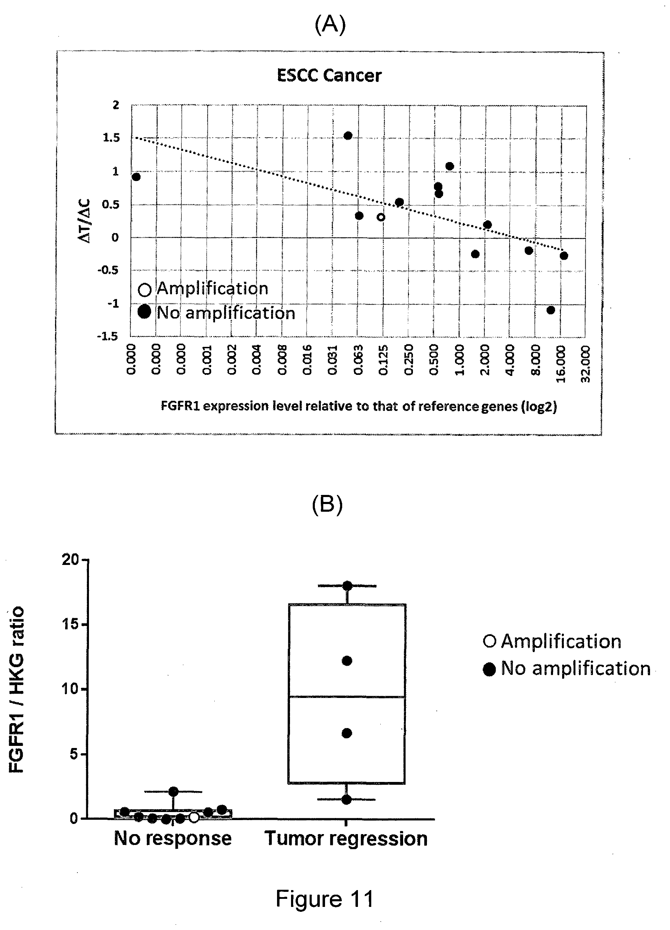

Figure 11 shows the relationship between efficacies of treatment (ΔT/ΔC) with Compound A and levels of expression of FGFR1 in PDX models of ESCC while indicating FGFR1 amplification (empty circles) as per Example 2. (A) correlation plot; (B) box plot.

### DETAILED DESCRIPTION OF THE INVENTION

### Definitions:

Compound A is 5-amino-1-(2-methyl-1H-benzimidazol-5-yl)-1H-pyrazol-4-yl]-(1H-indol-2-yl)-methanone (CAS 1265229-25-1). The compound was described in international patent application publication WO 2011016528 and Nakanishi, Y. et al. (2014) Mol. Cancer Ther. 13: 2547-58. It also includes pharmaceutically acceptable salts of such compound, including but not limited to acetic acid, adipic acid, L-ascorbic acid, L-aspartic acid, capric acid (decanoic acid), carbonic acid, citric acid, fumaric acid, galactaric acid, D-glucoheptonic acid, D-gluconic acid, D-glucuronic acid, glutamic acid, glutaric acid, glycerophosphoric acid, glycolic acid, hippuric acid, hydrochloric acid, DL-lactic acid, lauric acid, maleic acid, (-)-L-malic acid, palmitic acid, phosphoric acid, sebacic acid, stearic acid, succinic acid, sulfuric acid, (+)-L-tartaric acid and thiocyanic acid, where (-)-L-malic acid is preferred.

"FGFR" refers to a member of the family of fibroblast growth factor receptors. The FGFR family is a member of the receptor tyrosine kinase family. Four members of the FGFR family are known, i.e., FGFR1, FGFR2, FGFR3 and FGFR4. The FGFR as referred to in the present invention may be from any origin, but preferably from a mammalian and, more preferably, from a human origin. The present invention is concerned with FGFR1, 2 and/or 3, and the term FGFR as used herein refers to any or any combination of the latter three FGFR species.

"Cancer" generally refers to malignant neoplasm, which may be metastatic or non-metastatic. For instance, non-limiting examples of cancer that develops from epithelial tissues such as gastrointestinal tract and skin include brain tumor, skin cancer, head and neck cancer, esophageal cancer, lung cancer, stomach cancer, duodenal cancer, breast cancer, prostate cancer, cervical cancer, cancer of uterine body, pancreatic cancer, liver cancer, cholangiocarcinoma, gallbladder cancer, colorectal cancer, colon cancer, bladder cancer, and ovarian cancer. Non-limiting examples of sarcoma that develops from non-epithelial tissues (stroma) such as muscles include osteosarcoma, chondrosarcoma, rhabdomyosarcoma, leiomyosarcoma, liposarcoma, and angiosarcoma. Furthermore, non-limiting examples of hematological cancer derived from hematopoietic organs include malignant lymphoma including Hodgkin's lymphoma and non-Hodgkin's lymphoma, leukemia including acute myelocytic leukemia, chronic myelocytic leukemia, acute lymphatic leukemia, chronic lymphatic leukemia, and multiple myeloma. The latter examples of cancer are also referred to herein as types of cancer.

By a "therapeutically effective amount" of Compound A (also referred to herein as the "active agent" or the "compound") is meant an amount of the compound which, subsequent to single or multiple administration, confers a therapeutic effect on the treated subject, at a reasonable benefit/risk ratio applicable to any medical treatment. However, it is understood that effective doses will also vary depending on route of administration, as well as the possibility of co-usage with other agents, including anti-neoplastic agents. It will be understood, however, that the total daily usage of the compositions of the present invention will be decided by the attending physician within the scope of sound medical judgment. The specific therapeutically effective dose level for any particular patient will depend upon a variety of factors including the severity of the condition/disease; the specific composition employed; the age, body weight, general health, sex and diet of the patient; the time of administration, the route of administration, the rate of excretion of the active agent, the duration of the treatment; drugs used in combination or contemporaneously with the active agent, and like factors well known in the medical arts.

The term "pharmaceutically acceptable carrier" or "pharmaceutically acceptable vehicle" encompasses any of the standard pharmaceutical carriers, solvents, surfactants, or vehicles. Suitable pharmaceutically acceptable vehicles include aqueous vehicles and non-aqueous vehicles. Standard pharmaceutical carriers and their formulations are described, in a non-limiting fashion, in Remington's Pharmaceutical Sciences, Mack Publishing Co., Easton, PA, 19th ed. 1995.

The term "subject" relates to a mammalian animal and, preferably, to a human person.

The term "overexpression" relating to an FGFR means a level of expression (measured at the RNA or the protein level) in a tumor or a tumor biopsy that exceeds the level measured in the corresponding non-tumorous cells or tissue.

### Methodology:

Sensitivity to treatment with FGFR kinase inhibitor Compound A was examined in 66 patient-derived xenograft (PDX) models. PDX models were selected based on available information that had been obtained by RNA sequencing (RNA-Seq) using next-generation sequencing (NGS) technology and hybridization to an Affymetrix Human Genome U219 array plate. A balanced set of models was selected which had the following characteristics: (i) an increased copy number of an FGFR gene and overexpression of an FGFR, or (ii) an increased copy number of an FGFR gene without overexpression of an FGFR, or (iii) no increase in copy number of an FGFR gene but overexpression of an FGFR, or (iv) expression of an FGFR fusion gene product.

For each tumor model, the effects of Compound A on tumor growth (treatment efficacy), FGFR copy number and FGFR mRNA levels were assessed (or re-assessed). In several model studies, FGFR protein levels were also estimated using immunohistochemical methods. In a typical experiment, tumor fragments from seed mice inoculated with selected PDX tumors were harvested and used for inoculation of female Balb/c nude mice. Each mouse was inoculated subcutaneously at the right flank with one tumor fragment (2-3 mm in diameter) for tumor development. Treatments were started when mean tumor size reached approximately 200-250mm³. Compound A (60-80 mg/kg) formulated as a suspension in 1% Kollidon VA64 in deionized water or vehicle alone (i.e., 1% Kollidon VA64 in deionized water) were administered orally by gavage to the tumor-bearing mice daily for 14 consecutive days.

Tumor volume was measured twice weekly in two dimensions using a caliper, and the volume was expressed in mm³ using the formula: V = 0.5 a x *b*² where *a* and *b* are the long and short diameters of the tumor, respectively. Body weight was also recorded twice weekly. Treatment efficacy was expressed as ΔT/ΔC, whereby ΔT reports the relative change in tumor volume of drug-treated animals and ΔC the relative change in tumor volume of not-drug-treated animals between the last day of treatment and the beginning of treatment (median volume differences). Results obtained for the different models are shown in Table 2.

FGFR copy number was estimated by FISH. Tumor tissue obtained from tumor-bearing animals was fixed in buffered formalin and embedded in paraffin blocks (FFPE). Three to four µm tissue sections were mounted on silanized slides, deparaffinated, protease-treated, washed, DNA denatured and then hybridized to an FGFR probe as described by Schildhaus, H.U. et al. (2012) Mol. Pathol. 25: 1473-80. Subsequent to hybridization, tumor tissue was scanned for amplification hotspots using a fluorescent microscope as also described in Schildhaus et al. (2012). FGFR probes used were FGFR1/CEN 8 Dual Color Probe, FGFR2/CEN 10 Dual Color Probe and FGFR3/4p11 Dual Color Probe (all from Zytovision GmbH, Bremerhaven, Germany). Gene copy number gain (GCN) was defined as either amplification (FISH FGFR probe-centromere probe ratio ≥2.2) or polysomy, defined as FGFR probe-centromere probe ratio <2.2 but each one of FGFR and centromeres probes >2.

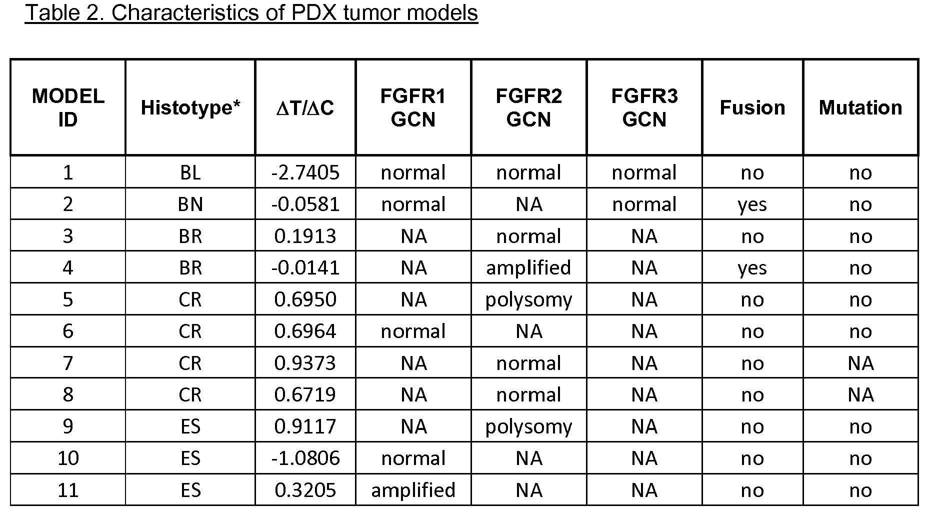

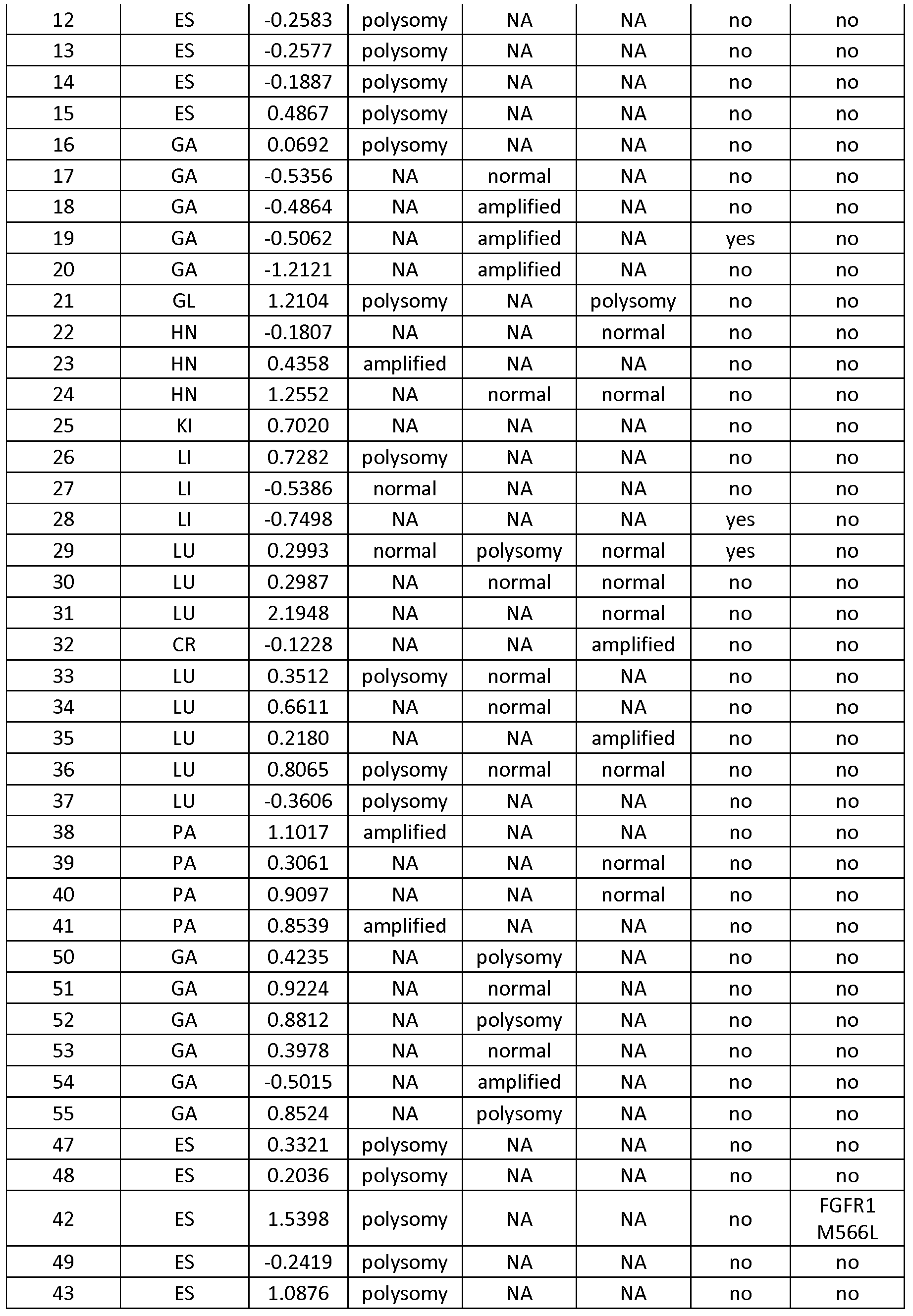

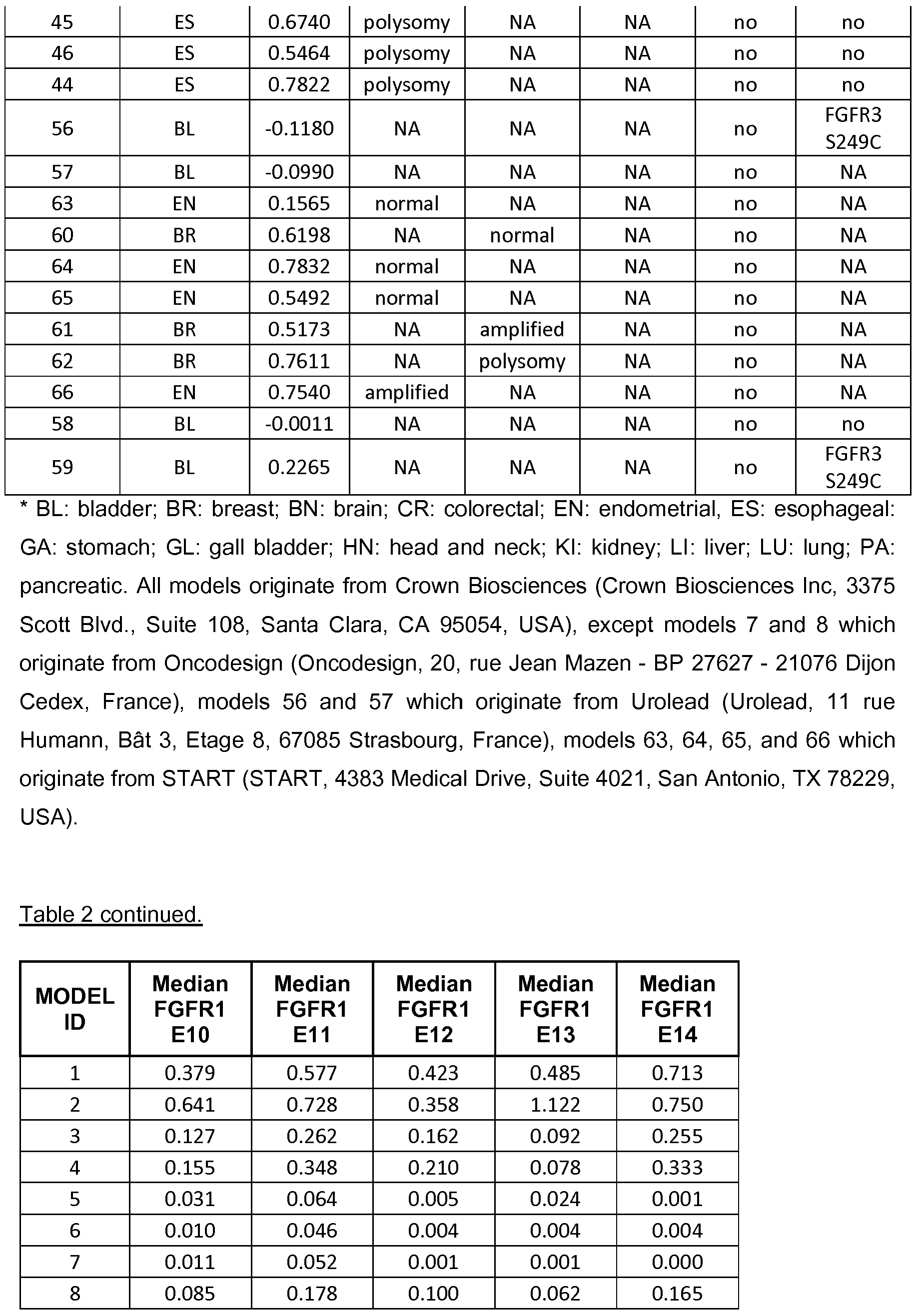

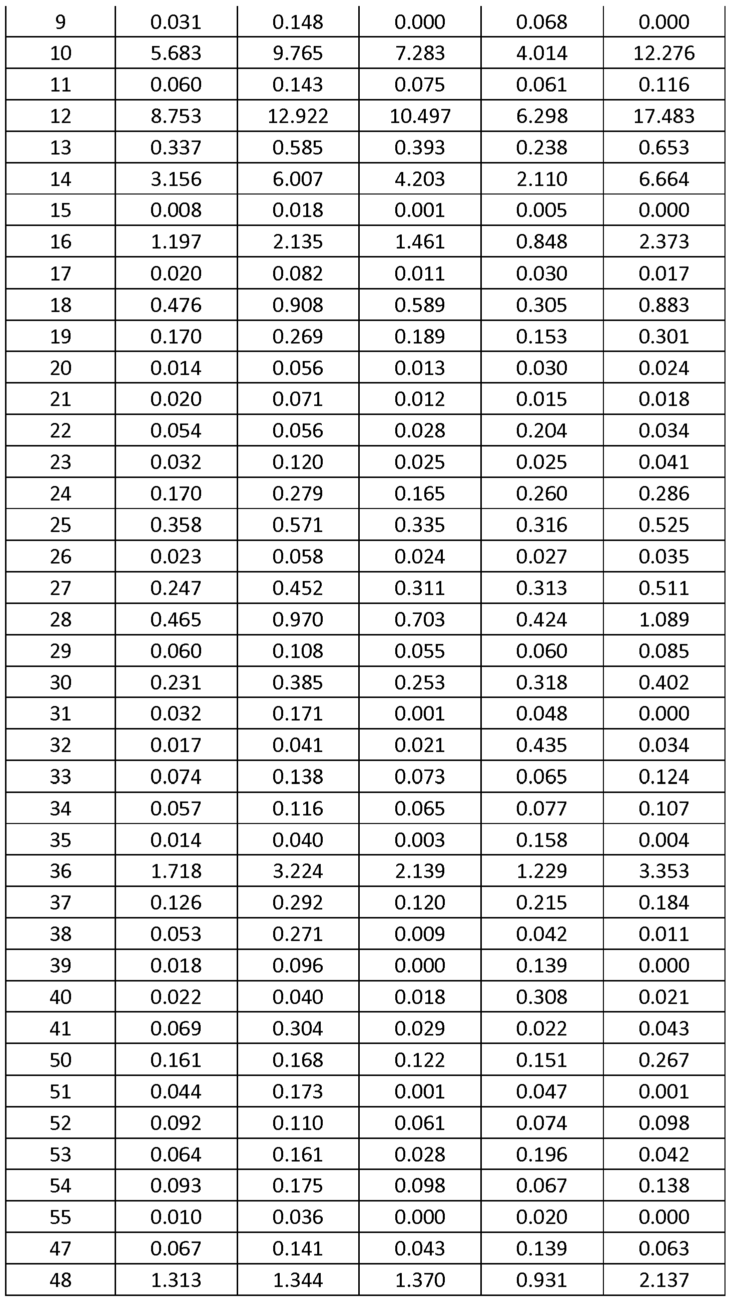

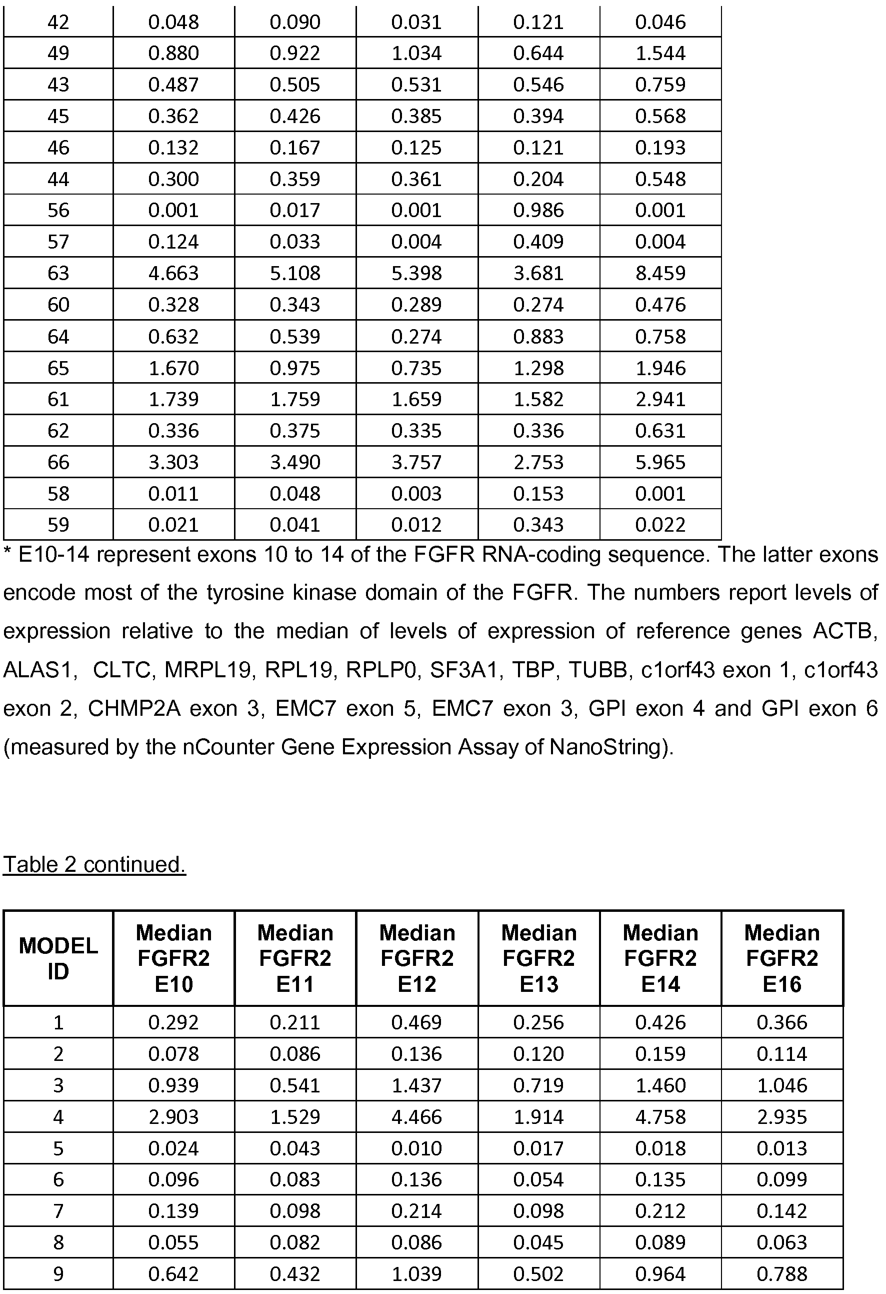

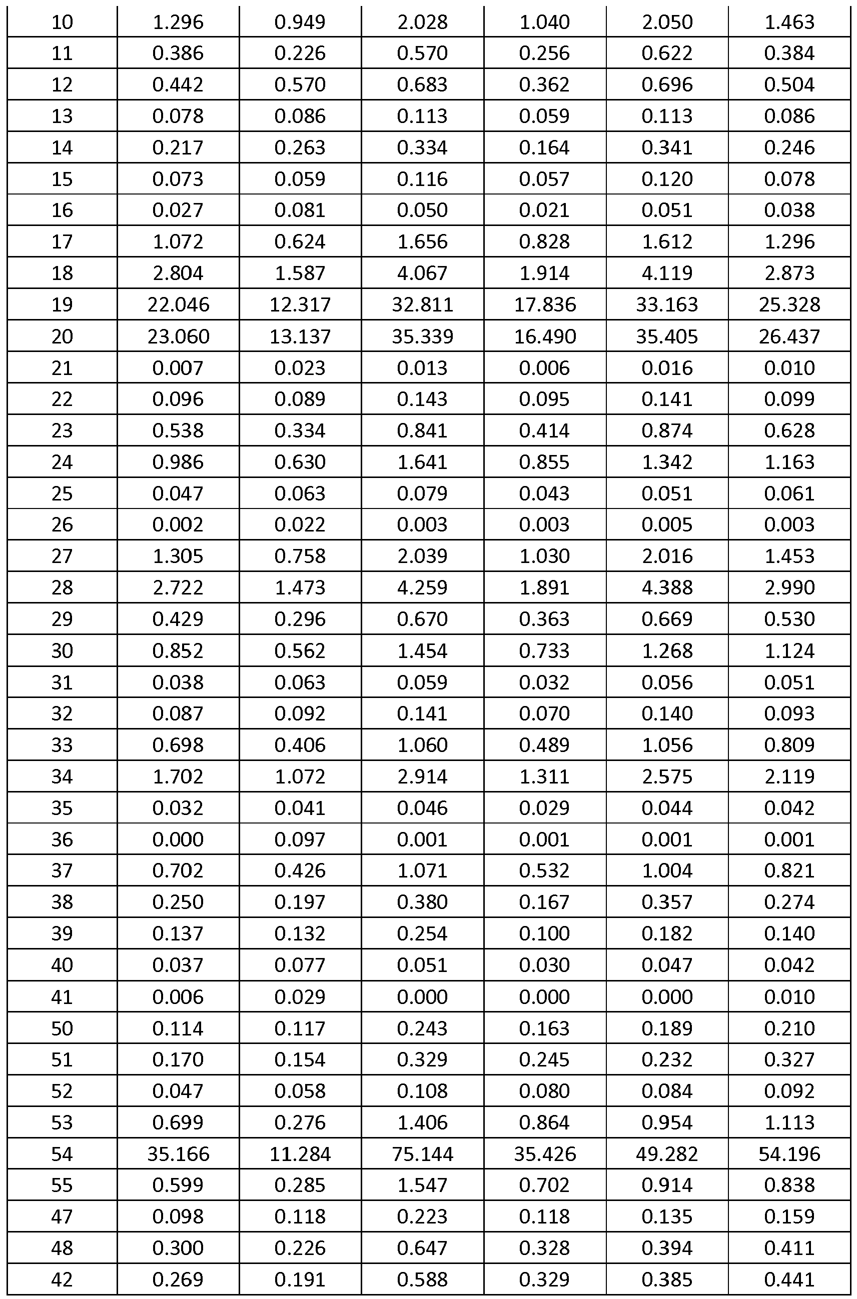

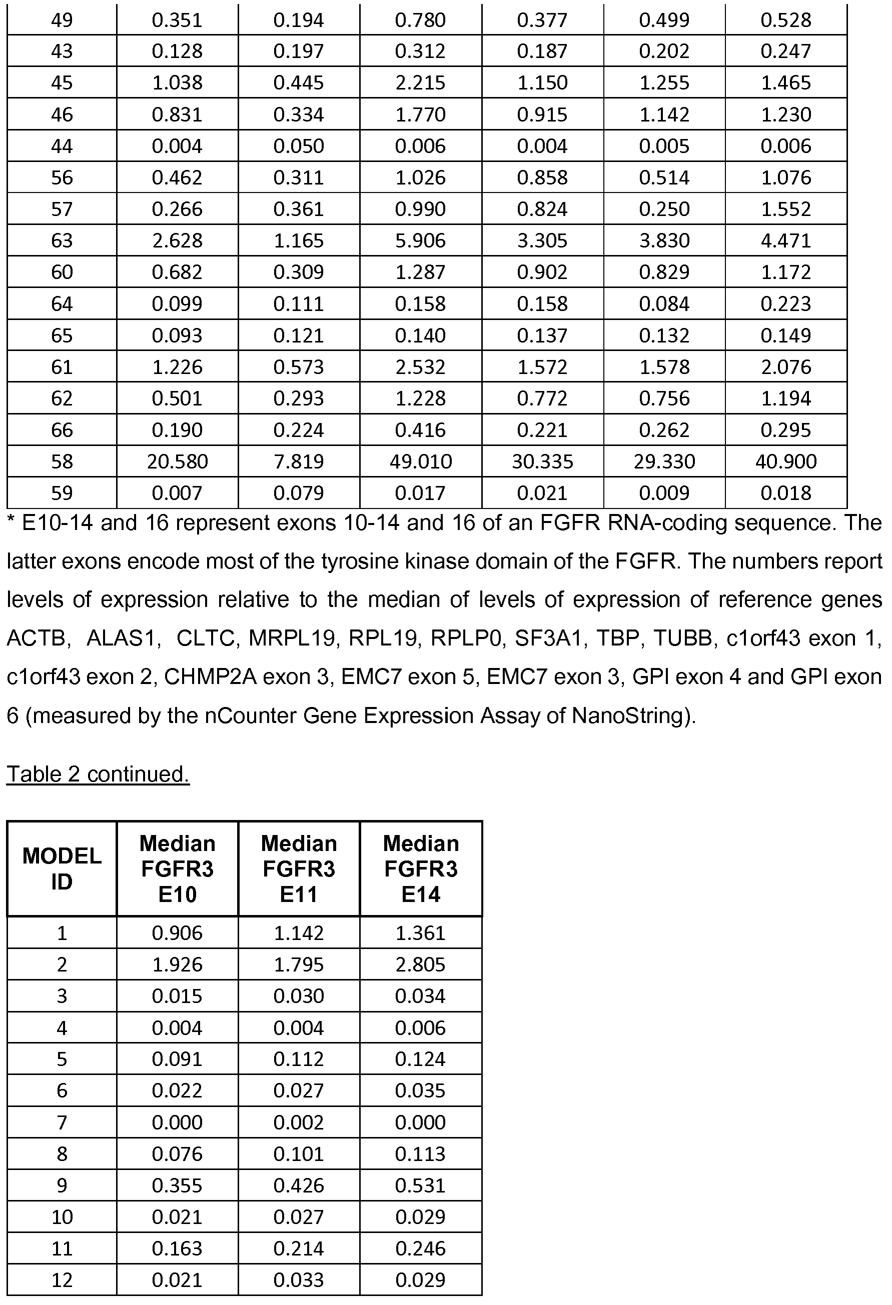

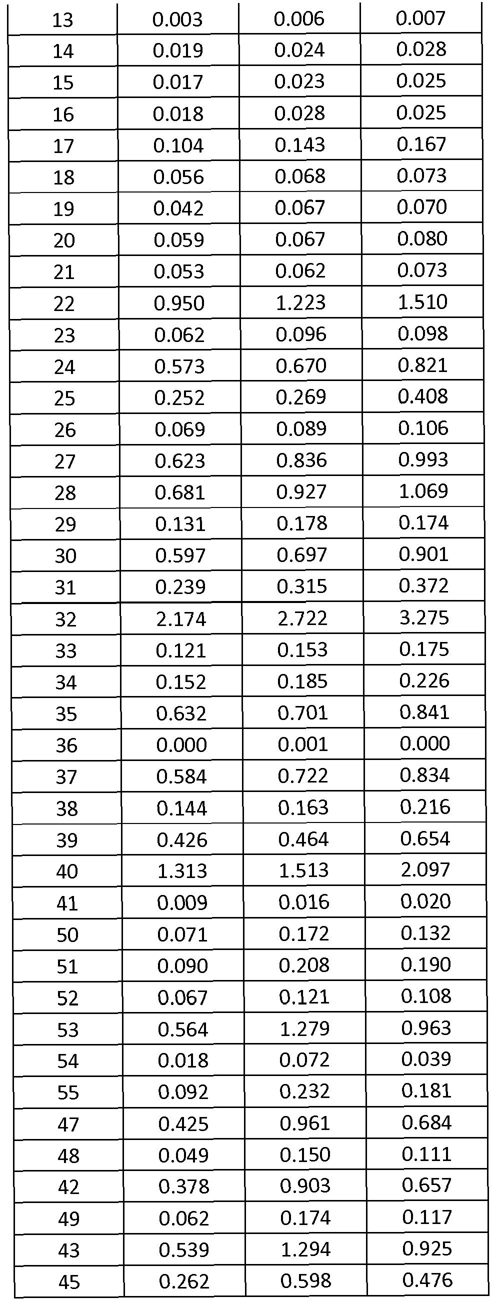

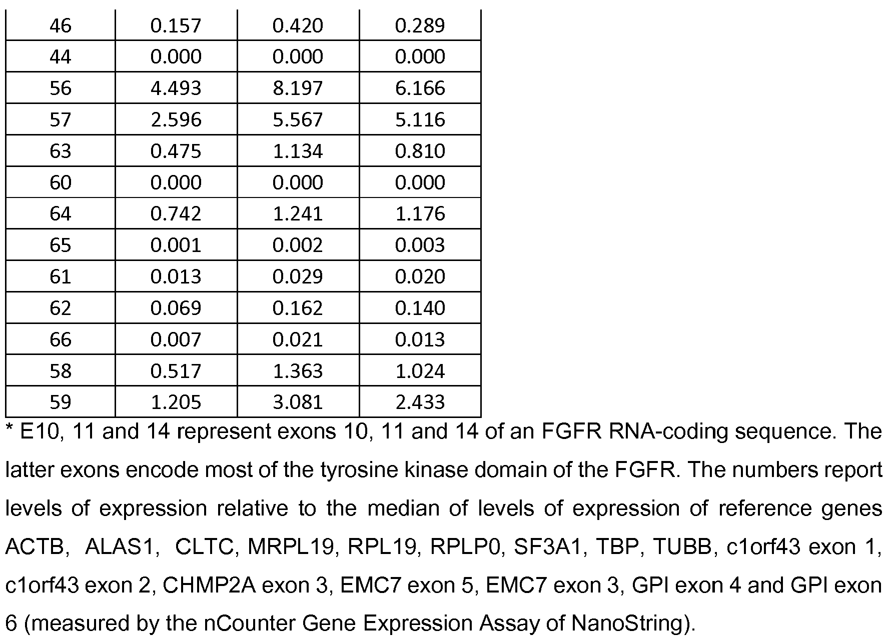

To determine FGFR mRNA levels, total RNA was extracted from one to six macro-dissected 10 µm thick FFPE sections using the QIAgen miRNeasy FFPE kit (QIAgen 217504) according to the manufacturer's instructions. The procedure involved lysis of the deparaffinized tissue using the QIAgen proprietary RNA tissue lysis buffer and incubation with Proteinase K. In the presence of chaotropic salts, the RNA was specifically bound to the glass fibers of a spin column. Bound RNA was incubated with DNase and purified in a series of rapid wash-and-spin steps and then eluted in water. RNA concentration was determined by absorbance using a NanoDrop spectrophotometer (Thermo Fischer Scientific, Waltham, MA, USA) and fluorescence using Qubit fluorometer (Life Technologies).

300 ng of total RNA per sample were analyzed using the nCounter Gene Expression Assay protocol as instructed by the manufacturer NanoString Technologies Inc., Seattle, WA, USA (www.nanostring.com). The nCounter assay is based on direct digital detection of mRNA molecules of interest using target-specific, color-coded probes that hybridize directly to the target molecules in solution, so that the expression level of each gene is measured in a relative fashion by counts, without the need for cDNA synthesis and amplification. Each probe is constituted of a reporter probe part of 50 bases that carries the barcode on the 5' end and a capture probe part of 50 bases that carries a biotin molecule on the 3' end allowing the target-probe complex to be immobilized on a streptavidin-coated nCounter Cartridge for digital data collection (Counts) after washout of excess probes. Descriptions of the NanoString methodology are found in Geiss, G.K. et al. (2008) Nat. Biotech. 26: 317-325; Malkow, V.A. et al (2009) BMC Research Notes 2: 80; and Guancial, E.A. et al. (2014) Cancer Med. 3(4): 835-44.

FGFR genes and reference (i.e. housekeeping) genes were selected by the inventors. Probes for such FGFR and housekeeping genes were designed and synthetized by NanoString Technologies Inc. (Custom CodeSet), then inserted with all consumables and reagents (including 8 negative and 6 positive normalization probes provided by NanoString) in a ready-to-use nCounter Master Kit for sample processing in the nCounter Analysis System. Negative normalization probes are used in the background correction (see below) and positive normalization probes are used for cartridge quality control only. The method of normalization using the positive normalization probes was that described in the nCounter Expression Data Analysis Guide published by NanoString in 2012. The target sequences (FGFR target sequences as well as target sequences for standardization, i.e. sequences of housekeeping genes) used for the design of probes, as provided by NanoString, are reproduced in Table 3 below. References for normalization probes are provided in Table 4 below. FGFR mRNA levels (expressed relative to those of a set of 16 reference genes consisting of ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6) determined for the different models are presented in Table 2.

Count values obtained were background-corrected. Background-corrected values were standardized based on parallel measurement of expression levels of "housekeeping genes" in each RNA specimen analyzed. The following procedure was used:

### Background correction

1. For each sample the background correction value was derived by calculating the mean + 2 times the standard deviation of the counts of the negative normalization probes.
2. The background-corrected values were obtained by subtracting the background correction value to the counts of all probes except the negative normalization probes.

### Housekeeping Gene Standardization

1. For each sample the median of the background-corrected standardization (housekeeping genes) probe counts was calculated [HKG MEDIAN].
2. The standardized counts were obtained by dividing the background-corrected counts of the FGFR probe by the HKG MEDIAN of the respective sample.

**Table 3: Target sequences used for the design of probes in the nCounter assay.**

| A. FGFR gene targets: | | | |
|---|---|---|---|
| Gene | Accession # | Target region | Target Sequence (SEQ ID NO.) |
| FGFR1 E10 | NM_015850.3 | 2222-2321 | |
| FGFR1 E11 | NM_015850.3 | 2384-2483 | |
| FGFR1 E12 | NM_015850.3 | 2491-2590 | |
| FGFR1 E13 | NM_015850.3 | 2703-2802 | |
| FGFR1 E14 | NM_015850.3 | 2811-2910 | |
| | | | |
| FGFR2 E10 | NM_000141.4 | 1921-2020 | |
| FGFR2 E11 | NM_000141.4 | 2083-2182 | |
| FGFR2 E12 | NM_000141.4 | 2205-2304 | |
| FGFR2 E13 | NM_000141.4 | 2409-2508 | |
| FGFR2E 14 | NM_000141.4 | 2527-2626 | |
| FGFR2 E16 | NM_000141.4 | 2705-2804 | |
| | | | |
| FGFR3 E10 | NM_000142.4 | 1533-1632 | |
| FGFR3 E11 | NM_000142.4 | 1702-1801 | |
| FGFR3 E14 | NM_000142.4 | 2111-2210 | |

| B. Reference gene targets ("housekeeping" genes used for standardization): | | | |
|---|---|---|---|
| Gene | Accession # | Target region | Target Sequence |
| ABCF1 | NM_001090.2 | 851-950 | |
| ACTB | NM_001101.2 | 1011-1110 | |
| ALAS1 | NM_000688.4 | 1616-1715 | |
| B2M | NM_004048.2 | 26-125 | |
| CLTC | NM_004859.2 | 291-390 | |
| G6PD | NM_ 000402.2 | 1156-1255 | |
| GAPDH | NM_002046.3 | 973-1072 | |
| GUSB | NM_000181.1 | 1351-1450 | |
| HPRT1 | NM_000194.1 | 241-340 | |
| LDHA | NM_001165414.1 | 1691-1790 | |
| MRPL19 | NM_014763.3 | 365-464 | |
| PGK1 | NM_000291.2 | 1031-1130 | |
| POLR1 B | NM_019014.3 | 3321-3420 | |
| POLR2 A | NM_000937.2 | 3776-3875 | |
| PSMC4 | NM_006503.2 | 251-350 | |
| RPL19 | NM_000981.3 | 316-415 | |
| RPLPO | NM_001002.3 | 251-350 | |
| SDHA | NM_004168.1 | 231-330 | |
| SF3A1 | NM_001005409.1 | 236-335 | |
| TBP | NM_001172085.1 | 588-687 | |
| TUBB | NM_178014.2 | 321-420 | |
| c1orf43 exon 1 | NM_015449.2 | 352-451 | |
| c1orf43 exon 4 | NM_015449.2 | 629-728 | |
| c1orf43 exon 2 | NM_015449.2 | 478-577 | |
| CHMP2 A exon 1 | NM_014453.3 | 242-341 | |
| CHMP2 A exon 3 | NM_014453.3 | 513-612 | |
| CHMP2 A exon 2 | NM_014453.3 | 397-496 | |
| EMC7 exon 5 | NM_020154.2 | 612-711 | |
| EMC7 exon 3 | NM_020154.2 | 403-502 | |
| GPI exon 4 | NM_000175.3 | 416-515 | |
| GPI exon 6 | NM_000175.3 | 612-711 | |

**Table 4. Positive and negative normalization probes***

| Code Class | Name | Accession # |
|---|---|---|
| Positive | POS_A(128) | ERCC_00117.1 |
| Positive | POS_B(32) | ERCC_00112.1 |
| Positive | POS_C(8) | ERCC_00002.1 |
| Positive | POS_D(2) | ERCC_00092.1 |
| Positive | POS_E(0.5) | ERCC_00035.1 |
| Positive | POS_F(0.125) | ERCC_00034.1 |
| Negative | NEG_A(0) | ERCC_00096.1 |
| Negative | NEG_B(0) | ERCC_00041.1 |
| Negative | NEG_C(0) | ERCC_00019.1 |
| Negative | NEG_D(0) | ERCC_00076.1 |
| Negative | NEG_E(0) | ERCC_00098.1 |
| Negative | NEG_F(0) | ERCC_00126.1 |
| Negative | NEG_G(0) | ERCC_00144.1 |
| Negative | NEG_H(0) | ERCC_00154.1 |

| | | |
|---|---|---|
| *NanoString provides the following additional information on negative and positive control normalization: The External RNA Control Consortium (ERCC) is a group of industry representatives established to develop RNA control transcripts that can be used to assess technical performance in gene expression assays. NanoString has adopted sequences developed and tested by the ERCC for positive and negative hybridization controls. The ERCC control sequences are not homologous to any known organism, are applicable and transferable in all CodeSets, and generate consistent results in gene expression analyses. Reporter probes designed against ERCC transcript sequences are pre-mixed into every CodeSet, and are therefore available for use in data analyses.* | | |

It is noted that normalized threshold values are sensitive to the reference gene set utilized for standardization (Table 5).

**Table 5. Normalized threshold values calculated with sets of 31, 16 or 3 housekeeping genes (HKG) for 41 models (70 percentile cutoff)**

| | FGFR1 | FGFR2 | FGFR3 |
|---|---|---|---|
| | E14 | E14 | E14 |
| 31 HKG | 0.67081839 | 1.73074441 | 0.58355056 |
| 16 HKG | 0.40163985 | 1.05557373 | 0.53066262 |
| 3 HKG | 0.98903303 | 2.12732335 | 1.11414638 |

Alternatively, FGFR mRNA levels may be determined by any other method capable of quantitating reliably mRNA levels. Suitable methods include but are not limited to:
(1) Northern blots. Northern blots can be utilized to estimate the molecular weight of an mRNA and to measure relative amounts of mRNAs present in different samples. A general blotting procedure starts with extraction of total RNA from a homogenized tissue sample or from cells. RNA is separated by gel electrophoresis, usually on an agarose gel. Because there are so many different RNA species on the gel, RNA usually appears as a smear rather than discrete bands. The RNA is transferred to a nitrocellulose membrane, although other types of membranes can also be used. The RNA molecules retain the same pattern of separation they had in the gel. The blot is incubated with a probe, typically a single-stranded DNA. This probe will form base pairs with its complementary RNA sequence and bind to form a double-stranded RNA-DNA molecule. The probe may be radiolabeled or coupled to an enzyme (e.g., alkaline phosphatase or horseradish peroxidase). In the latter case, the location of the probe is revealed by incubating it with a colorless substrate that the attached enzyme converts to a colored product that can be seen or gives off light which will expose X-ray film. If the probe was labeled with radioactivity, it can expose X-ray film directly. (2000) Invest. Ophthalmol. Vis. Sci. 41: 2357-62.
(2) Nuclease protection assays (NPA). The NPA (including both ribonuclease protection assays and S1 nuclease assays) is an extremely sensitive method for the detection and quantitation of specific mRNAs. The extracted RNA is first mixed with radiolabeled RNA or DNA probes that are complementary to the sequence or sequences of interest and the complementary strands are hybridized to form double-stranded RNA (or DNA-RNA hybrid). The mixture is then exposed to ribonucleases that specifically cleave only single-stranded RNA (DNA) but have no activity against double-stranded RNA (or DNA-RNA hybrid). When the reaction runs to completion, susceptible RNA regions are degraded to very short oligomers or to individual nucleotides; the surviving RNA fragments are those that were complementary to the added antisense strand and thus contained the sequence of interest. The remaining protected fragments are separated by gel electrophoresis and visualized by autoradiography. Maddula, K. et al. FGFR and FGF ligand overexpression in lung cancer: Implications for targeted therapy. HTG Molecular Diagnostics, Tucson, AZ, USA, 2014 ASCO Annual Meeting.
(3) Reverse transcription - quantitative polymerase chain reaction (RT-qPCR). RT-qPCR has revolutionized the study of gene expression. It is now theoretically possible to detect the RNA transcript of any gene, regardless of the scarcity of the starting material or the relative abundance of the specific mRNA. In RT-qPCR, an RNA template is copied into a complementary DNA (cDNA) using a retroviral reverse transcriptase. The cDNA is then amplified exponentially by PCR and the amplification is linked to the generation of fluorescence which can simply be detected with a camera during each PCR cycle. The process is monitored in "real-time" so is quantitative. Relative quantitative RT-qPCR involves amplifying an internal control transcript simultaneously with the gene transcript of interest. The internal control is used to standardize the samples. Once standardized, direct comparisons of relative abundance of a specific mRNA can be made across the samples. For relative RT-qPCR results to be meaningful, all products of the PCR reaction must be analyzed in the linear range of amplification. Yan, D., et al. (2011) Arthritis Res. Ther. 13(4): R130; Wong, M.L. and Medrano, J.F. (2005) BioTechniques 39: 75-85.
(4) Hybridization to DNA microarray. GeneChip microarrays use hybridization of DNA segments (on the array) and RNA target molecules (from the samples to be analyzed) to determine the expression level, i.e., how much RNA is being made from a given gene. Known segments of DNA are used as "probes" to bind and identify sample mRNA. Although this is not substantially different from other hybridization techniques (such as Southern and Northern blotting), what is unique about microarrays is the amount of information that is provided at one time. Tens of thousands of DNA sequences (or even larger numbers of oligonucleotides) are arranged in a known and orderly fashion (hence "array") on a small solid support structure or microchip. The RNA sample after amplification and labeling is allowed to bind to the array. If a gene in the sample is expressed, its mRNA (cDNA) will hybridize to its complementary DNA probe on the chip. The array is then scanned, providing both qualitative and quantitative information about the function of specific genes. Dalma-Weiszhausz, D.D. et al. (2006) Methods Enzymol. 410: 3-28; Yadav, V. et al. (2012) J. Biol. Chem. 287: 28087-98.
(5) RNA-Seq. RNA-Seq is a recently developed approach to transcriptome profiling that uses deep-sequencing technologies. RNA-Seq also provides a far more precise measurement of levels of transcripts and their isoforms than other methods. All RNA-Seq experiments follow a similar protocol. Total RNA is isolated from a sample of interest which, depending on the type of RNA to be profiled, may be purified to enrich for mRNAs, microRNAs or lincRNAs, etc., prior to preparing a library. Library preparation may involve such steps as reverse transcription to cDNA, PCR amplification and may or may not preserve strandedness information. Sequencing can produce one read in a single-end sequencing reaction, or two ends separated by an unsequenced fragment in paired-end reactions. Li, F. et al. (2015) Cancer Discov. 5: 438-51. For a review of deep sequencing technologies, see Metzker, M.L. et al. (2010) Nat. Rev. Genet. 11: 31-46.

Expression of FGFR genes may also be assessed by methods that are capable of quantitating reliably the protein levels of different FGFRs. Suitable methods include but are not limited to:
(1) Western blotting. Western blotting uses gel electrophoresis to separate native proteins by 3-D structure or denatured proteins by the length of the polypeptide. The proteins are then transferred to a membrane (typically nitrocellulose or PVDF), where they are stained with primary antibodies specific to the target protein. Once bound the antibody is visualized, either with a specific tag coupled to the primary antibody or with a secondary antibody that can be visualized. Wynes, M.W. et al. (2014) Clin. Cancer Res. 20: 3299-309.
(2) Immunodot assay. Dot blotting is a simple technique to identify a known protein in a biological sample. The ease and simplicity of the technique makes dot blotting an ideal diagnostic tool. The key feature of dot blotting is the use of immunodetection to identify a specific protein, for example a protein marker for a disease. Once proteins are immobilized on a protein-binding membrane, usually nitrocellulose or PVDF, they can be probed with a primary antibody, i.e., an antibody specific for the protein of interest. Once bound, the antibody is visualized either with a specific tag coupled to the primary antibody or with a secondary antibody. Girjes, A.A. et al. (1993) Veterinary Record 133: 136-41; Shekhar, M.S. (2010) Aquaculture Research 41: 1683-90.
(3) ImmunoAssay. The enzyme-linked immunosorbent assay (ELISA) is a test that uses antibodies and color change to identify a substance, and a solid-phase enzyme immunoassay (EIA) to detect the presence of a substance, usually an antigen, in a liquid sample or wet sample. Antigens from the sample are attached to a surface. Then, a further specific antibody is applied over the surface so it can bind to the antigen. This antibody is linked to an enzyme, and, in the final step, the enzyme's substrate is added. The subsequent reaction produces a detectable signal, most commonly a color change in the substrate. A number of platform technologies offer methods for multiplexed and miniaturized immunoaffinity assays (e.g., Luminex, MesoScale Discovery and PerkinElmer). Human fibroblast growth factor receptor 3 (FGFR3) ELISA Kit (http://www.cusabio.com/); Lequin, R.M. (2005) Clin. Chem. 51: 2415-8.
(4) Immunochromatography. Immunochromatography assay (also known as lateral flow or strip test) utilizes simple devices intended to detect the presence (or absence) of a target analyte in a sample (matrix) without the need for specialized and costly equipment, though many applications exist that are supported by reading equipment. The technology is based on a series of capillary beds, such as porous paper or sintered polymer. Each of these elements has the capacity to transport fluid (e.g., urine) spontaneously. The first element (the sample pad) acts as a sponge and holds an excess of sample fluid. Once soaked, the fluid migrates to the second element (conjugate pad), a dried format of bioactive particles in a salt-sugar matrix that contains everything to guarantee an optimized chemical reaction between the target molecule (e.g., an antigen) and its chemical partner (e.g., antibody) that has been immobilized on the particles' surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes color. Typically, there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material, the wick that simply acts as a waste container. Zeng. Q. et al. (2009) Am. J. Biomed. Sci. 1: 70-79.
(5) Immunohistochemistry. Immunohistochemistry (IHC) is a method capable of demonstrating the presence and location of proteins in tissue sections. Though being semi-quantitative only, it enables the observation of processes in the context of intact tissue. The basic steps of the IHC protocol are as follows: fixing and embedding the tissue, cutting and mounting the section, deparaffinizing and rehydrating the section, applying antigen retrieval process, immunohistochemical staining and viewing the staining under the microscope. The following protocol was used to detect FGFR expression in tumor tissues: immunostaining was performed on 4-µm paraffin-embedded tissue sections. Briefly, slides were deparaffinized in xylene and dehydrated utilizing a graded ethanol series, and endogenous peroxidase was blocked with 3% hydrogen peroxide. After epitope retrieval, the slides were washed with and blocked with TRIS-buffered saline with 0.1% (vol.) Tween 20/5% (vol.) normal goat serum. Incubation with the primary antibody was performed overnight at 4°C followed by incubation with the secondary antibody for 30 min at room temperature. Sections were washed three times with TRIS-buffered saline with 0.1% (vol.) Tween 20, stained with diaminobenzidine (DAB) and counterstained with hematoxylin. Primary antibodies:
   FGFR1 Rabbit monoclonal antibody (Cell Signaling Technology, Cat# 9740, clone #D8E4)
   FGFR2 Rabbit polyclonal antibody (Novus Biologicals, Cat# NB200-642)
   FGFR3 Mouse monoclonal antibody (Santa Cruz Biotechnology, Cat# sc-13121, clone B-9)
   Guancial et al. (2014); Redler, A. et al. (2013) PLoS One. 8: e72224.
(6) Mass Spectrometry. Mass spectrometry (MS) measures the mass-to-charge ratio of ions to identify and quantify proteins. While mass spectrometry can detect very low analyte concentrations in complex mixtures, MS is not inherently quantitative because of the considerable loss of peptides and ions during analysis. Therefore, peptide labels or standards are concomitantly analyzed with the sample and act as a reference point for relative or absolute peptide quantitation. Catenacci. D.V. et al. (2014) PLoS One 9: e100586; Razavi, M. et al. (2013) Clin. Chem. 59:1514-22. A mass spectrometry approach using Liquid Tissue-Selected reaction monitoring (LT-SRM) can be used to objectively quantify the levels of FGFR. Hembrough, T. et al. (2012) Clin. Proteomics: 9: 5; Hembrough, T. et al. (2013) J. Mol. Diagn. 5:454-65.
(7) Flow Cytometry. Flow cytometry is a laser-based, biophysical technology employed in cell counting, cell sorting, biomarker detection and protein engineering, involving suspending cells in a stream of fluid and passing them by an electronic detection apparatus. It allows simultaneous multiparametric analysis of the physical and chemical characteristics of up to thousands of particles per second. Using antibody specific of protein, flow cytometry can provide information regarding the expression of cell surface and, in some cases, cytoplasmic or nuclear markers that are used to understand complex cellular populations or processes. Yan, D. et al. (2011) Arthritis Res.Ther. 13: R130.

A waterfall plot of the ΔT/ΔC values of all 66 PDX models is shown in Figure 1. When a ΔT/ΔC value of 0 was used to define efficacy of drug treatment (complete inhibition of tumor growth), only 21 of 66 tumors (response rate of about 32%) were effectively treated by Compound A. Since the PDX models are low-passage models of human tumors and, therefore, are likely to closely reproduce the properties of human tumors, the data were taken to predict that only about 32% of human subjects (who had an amplified FGFR gene, expressed an FGFR fusion gene product and/or exhibited some level of overexpression of an FGFR mRNA) will respond to the drug treatment and that nearly 68% of subjects will likely not benefit from the drug. It is noted that typical criteria for selection of human subjects for trials of selective tyrosine kinase inhibitors (New Chemical Entities) that have anti-FGFR activity are an increased gene copy number (amplification, polysomy) of an FGFR gene or the presence of an FGFR fusion protein gene. A waterfall plot of the ΔT/ΔC values of all PDX models that exhibited a gene copy number gain and/or the presence of a FGFR fusion gene also revealed a response rate of 32.5% (Figure 2, right plot). Therefore, application of these selection criteria failed to identify the subgroup of models that are effectively treated by Compound A or to at least enrich for this subgroup. Essentially the same result was obtained from the converse analysis, i.e. when all PDX models were considered for which no gain in gene copy number for any FGFR or expression of an FGFR fusion gene had been found (Figure 2, left plot).

Surprisingly, when only models were considered that overexpressed an FGFR to a certain level, 21 of 40 PDX models (response rate of about 52%) were found to be responsive to Compound A (Figure 3, right plot). Conversely, when only models that did not overexpress any FGFR to that level were included in the analysis, the response rate was 0 (Figure 3, left plot). These findings strongly suggested that overexpression of an FGFR (i.e., FGFR1, FGFR2 and FGFR3) is a considerably better predictor of successful therapy with Compound A than an increased copy number of an FGFR gene and/or the presence of an FGFR fusion gene. For the purposes of the latter analysis an FGFR was considered significantly overexpressed if its standardized RNA level was higher than that found in 73% of the 66 PDX models (73^{th} percentile cutoff level of expression). The threshold levels (FGFR Exon 14 RNA/reference RNA determined by the NanoString technology as described above) were 0.641 for FGFR1, 1.094 for FGFR2 and 0.815 for FGFR3.

A post-hoc analysis of all models identified the presence of FGFR mutations in some models. Calculation of the response rates including such mutations as a selection criteria surprisingly confirmed the superior predictive power of FGFR overexpression over genetic alterations. A waterfall plot of ΔT/ΔC values of all PDX models that exhibited a gene copy number gain and/or the presence of a FGFR fusion gene and/or an FGFR mutation revealed a response rate of about 32% (Figure 4, right plot). Therefore, similarly as above, application of genetic alterations as selection criteria including FGFR mutations also failed to identify the subgroup of models that are effectively treated by Compound A or to at least enrich for this subgroup. Essentially the same result was obtained from the converse analysis, i.e., when all PDX models were considered for which neither a gain in gene copy number for any FGFR, expression of an FGFR fusion gene or presence of an FGFR mutation had been found (Figure 4, left plot).

It is noted that the predictive power of FGFR overexpression can be altered by decreasing or increasing the threshold FGFR expression levels. At the 44^{th} percentile cutoff level of expression, the response rate to therapy with Compound A was about 36% (Table 1). This is still a better response rate than that produced by selection for an increased copy number of an FGFR gene and/or the presence of an FGFR fusion gene and/or the presence of an FGFR mutation. At the 73^{th} percentile cutoff level of expression, the response rate was considerably higher, i.e., over 52% (Figure 3, right plot; Table 1). At the 90^{th} percentile cutoff, it was over 71%. However, at the 74^{th} percentile and higher cutoff levels, some models that respond to Compound A were excluded.

The overexpression selection criterion was found to be relatively insensitive to the ΔT/ΔC threshold value that was chosen to separate effective from ineffective treatment by Compound A. In the above analysis, the ΔT/ΔC threshold was set at zero (0), corresponding to complete arrest of tumor growth during the treatment period. When the ΔT/ΔC threshold was set at 0.4, corresponding to substantial reduction of tumor growth during the treatment period, the response rate without selection was 51.5% (Figure 5). When only models were considered that had an increased copy number of an FGFR gene and/or expressed an FGFR fusion gene, response rate was 50% (Figure 6, right plot). When only PDX models were considered for which no gain in gene copy number for any FGFR nor detectable expression of an FGFR fusion gene had been found, the response rate was 53.8% (Figure 6, left plot). These findings again suggested that the latter two selection criteria were of little or no predictive value. When only models were considered that overexpressed an FGFR (73^{th} percentile cutoff level of expression), the response rate reached over 72% (Figure 7, right plot). Thus, even using this less stringent threshold of 0.4 for treatment efficacy, overexpression of an FGFR still was a significant predictor of successful therapy with Compound A.

With a ΔT/ΔC threshold set at 0.4, the same observation as above can be made when considering FGFR mutations in the selection criteria. When only models were considered that had an increased copy number of an FGFR gene, expressed an FGFR fusion gene and/or had a mutation in an FGFR gene, response rate was 52.4% (Figure 8, right plot). When only PDX models were considered for which neither a gain in gene copy number for any FGFR, expression of an FGFR fusion gene or presence of an FGFR mutation had been found, the response rate was 50% (Figure 8, left plot).

As discussed previously, the above analyses were not limited by the methodology used to estimate FGFR expression. Analogous data could be produced by other methods of measuring mRNA levels such as Northern blots, nuclease protection assays, RT-qPCR, microarray hybridization or RNA-Seq. An example demonstrating that RT-qPCR and NanoString mRNA expression results are very well correlated is shown in Figure 9. The graphs show mRNA levels measured by each of RT-qPCR and NanoString technologies on test sets of PDX tumor samples: (A) 16 esophageal squamous-cell carcinoma (ESCC) PDX tumor samples (R² = 0.915 for FGFR1 mRNA levels) and (B) 26 gastric PDX tumor samples (R² = 0.957 for FGFR2 mRNA levels). NanoString measurements were performed following the process described in the Methodology section herein, except that standardization was performed using the same set of three reference genes that was employed for standardization of the RT-qPCR data (see below). RT-qPCR measurements were performed as follows: total RNA from FFPE tissue sections was isolated using the miRNeasy FFPE kit (QIAgen). Reverse transcription was carried out with 500ng RNA using the Transcriptor Universal cDNA Master (Roche). For qPCR, cDNA was amplified using FAM dye-labeled TaqMan Assays (Roche, Thermo) and the LightCycler® 1536 DNA Probes Master (Roche). Calculation of normalized relative expression levels was done using the qbasePLUS software version 3.0 (Biogazelle). Normalization was performed using three stably expressed reference genes (CLTC, ALAS1 and MRPL19) validated using the geNorm module in qbasePLUS. FGFR overexpression could also be assessed at the level of protein expression by methods such as Western blotting, dot blotting, ELISA, immunochromatography, immunohistochemistry, mass spectrometry and flow cytometry. Based on data obtained with any of these methods, the desired percentile cutoff and corresponding threshold values for FGFR mRNA or protein expression could be determined as discussed before.

Overexpression of an FGFR is a successful criterion for the selection of subjects that are likely to respond to therapy with Compound A independent of cancer indication or subtype. Stratification of the analysis based on cancer indication or subtype has the potential of enhancing the predictive power of the overexpression analysis (that is carried out as described above) in particular indications or subtypes. Examples showing that the correlation between FGFR expression and treatment efficacy holds for particular indications are presented in Figures 10 and 11. For example, the graph in Fig. 11A shows that in PDX models of esophageal squamous-cell carcinoma (ESCC) efficacy of treatment with Compound A increases with the degree of FGFR1 expression. The ΔT/ΔC ratio is inversely correlated with FGFR1 expression in the esophageal models studied (Spearman correlation p-value: 0.0043872).

Based on the above-described findings and conclusions, the inventors propose a method for identifying a cancer likely to respond to Compound A, consisting of 5-amino-1-(2-methyl-1H-benzimidazol-5-yl)-1H-pyrazol-4-yl]-(1H-indol-2-yl)-methanone. The first step consists in determining the levels of expression of FGFR1, FGFR2 and FGFR3 in a tumor tissue biopsy or a liquid biopsy (e.g., blood, from which circulating tumor cells or exosomes may be isolated). In the next step, amounts of FGFR1, FGFR2 and FGFR3 RNA are estimated. In the final step, the expression values so obtained are compared to the pre-established threshold values that were determined by analyses of the type discussed before. If a subject's level of RNA for any of FGFR1, FGFR2 and FGFR3 exceeds the respective threshold value, the subject is considered a candidate for treatment with Compound A, wherein levels of expression of FGFR1, FGFR2 and FGFR3 are measured at the messenger RNA level, and wherein the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

It is noted that pre-established threshold levels for specific cancer indications and, consequentially, cut-off levels, may be different from those determined for all indications. For esophageal squamous-cell carcinoma, threshold FGFR1 levels corresponding to:
(a) any levels corresponding to cut off levels of from about 44% to about 84% (0.104 - 1.362) may be selected as this range of threshold levels does not exclude any model that is capable of responding to Compound A, or
(b) any levels corresponding to cutoff levels from about 60% and about 84% (0.301 - 1.362), or
(c) any levels corresponding to cutoff levels from about 70% and about 84% (0.558 - 1.362), or
(d) any levels corresponding to cutoff levels from about 80% and about 84% (0.759 - 1.362).

The 16 reference genes being ACTB, ALAS1, CLTC, MRPL19, RPL19, RPLP0, SF3A1, TBP, TUBB, c1orf43 exon 1, c1orf43 exon 2, CHMP2Aexon 3, EMC7 exon 5, EMC7 exon 3, GPI exon 4 and GPI exon 6.

For gastric cancer, threshold FGFR2 levels corresponding to:
(a) any levels corresponding to cutoff levels of from about 44% to about 83% (0.257 - 1.610) may be selected as this range of threshold levels does not exclude any model that is capable of responding to Compound A, or
(b) any levels corresponding to cutoff levels from about 60% and about 83% (0.669 - 1.610), or
(c) any levels corresponding to cutoff levels from about 70% and about 83% (0.984 - 1.610), or
(d) any levels corresponding to cutoff levels from about 80% and about 83% (1.460 - 1.610).

The 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

It is noted that in the general (indication-independent) method, the levels of FGFR1, FGFR2 and FGFR3 are determined, whereas in a method relating to a specific type of cancer, it is sufficient to determine the level of the FGFR(s) of interest (i.e. known to be elevated in tumors of a fractions of subjects suffering from a cancer of this type) only.

It is further noted that the disclosed method relies on the rational assumption that a patient's tumor and a tumor grown in an animal directly from fragments of the patient's tumor exhibit closely similar levels of expression of FGFR genes at both RNA and protein levels. The plausibility of this assumption was borne out by a recent analysis by Guo et al. 2016 Cancer Res. [Epub ahead of print] (CAN-15-3245 Published June 2016).

Pharmaceutical compositions of the present invention comprising FGFR kinase inhibitor Compound A may be administered orally, parenterally, by inhalation spray, topically, rectally, nasally, buccally, vaginally or via an implanted reservoir, preferably by oral administration or administration by injection. The pharmaceutical compositions may contain any conventional non-toxic pharmaceutically acceptable carriers, adjuvants or vehicles. In some cases, the pH of the formulation may be adjusted with pharmaceutically acceptable acids, bases or buffers to enhance the stability of the active agent or its delivery form. Standard pharmaceutical carriers and their formulations are described, in a nonlimiting fashion, in Remington's Pharmaceutical Sciences, Mack Publishing Co., Easton, PA, 19th ed. 1995. The term parenteral as used herein includes subcutaneous, intracutaneous, intravenous, intramuscular, intraarticular, intraarterial, intrasynovial, intrasternal, intrathecal, intralesional and intracranial injection or infusion techniques.

Liquid dosage forms for oral administration include pharmaceutically acceptable emulsions, microemulsions, solutions, suspensions, syrups and elixirs. In addition to active agent, the liquid dosage forms may contain inert diluents commonly used in the art such as, for example, water or other emulsifiers, solubilizing agents and solvents such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions, may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride and Dextrose solutions. In addition, sterile, fixed oils are conventionally employed as a solvent or suspending medium. For this purpose, any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, autoclaving, dry heat, ionizing radiation, or by incorporating active agent in the form of a sterile solid composition which can be dissolved or dispersed in sterile water or other sterile injectable medium prior to use.

In order to prolong the effect of the active agent, it is often desirable to slow the absorption of the active agent from subcutaneous or intramuscular injection. This may be accomplished by the use of a liquid suspension of crystalline or amorphous material with poor water solubility. The rate of absorption of the active agent then depends upon its rate of dissolution, which, in turn, may depend upon crystal size and crystalline form. Alternatively, delayed absorption of a parenterally administered drug form is accomplished by dissolving or suspending the active agent in an oil vehicle. Injectable depot forms are made by microencapsulating the active agent in biodegradable polymers such as polylactide-polyglycolide. Depending upon the ratio of active agent to polymer and the nature of the particular polymer employed, the rate of release of the active agent can be controlled. Examples of other biodegradable polymers include poly(orthoesters) and poly(anhydrides). Depot injectable formulations are also prepared by entrapping the active agent in liposomes or microemulsions that are compatible with body tissues.

Solid dosage forms for oral administration include capsules, tablets, pills, powders and granules. In such solid dosage forms, active agent is mixed with at least one inert, pharmaceutically acceptable excipient or carrier such as sodium citrate or dicalcium phosphate and/or: a) fillers or extenders such as starches, lactose, cellulose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, croscarmellose, crospovidone, carboxymethylcellulise, potato or tapioca starch, alginic acid, certain silicates, and sodium carbonate, e) solution-retarding agents such as paraffin, f) absorption accelerators such as quaternary ammonium compounds, g) wetting agents such as, for example, cetyl alcohol, sodium lauryl sulfate and glycerol monostearate, h) absorbents such as kaolin and bentonite clay, and/or i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active agent only, or preferentially, in a certain part of the intestinal tract, optionally, in a delayed manner. Examples of embedding compositions that can be used include polymeric substances and waxes.

In general, treatment regimens according to the present invention comprise administration to a human subject in need of such treatment from 10 mg to 250 mg of Compound A per day in single or multiple doses, such as 2 daily doses.

The amount of active agent that may be combined with pharmaceutically acceptable excipients or carriers to produce a single dosage form will vary depending on the particular mode of administration and, possibly, on the subject treated. A typical preparation will contain from 1% to 95% active agent (w/w). Alternatively, such preparations may contain from 20% to 80% active agent. Lower or higher doses than those recited above may be required. Specific dosage and treatment regimens for any particular subject will depend upon a variety of factors, including the age, body weight, body surface area, general health status, sex, diet, time of administration, rate of excretion, drug combination, the severity and course of the disease, condition or symptoms, the subject's disposition to the disease, condition or symptoms, and the judgment of the treating physician.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e. g., all exact exemplary values provided with respect to a particular factor or measurement can be considered to also provide a corresponding approximate measurement, modified by "about," where appropriate).

The use of any and all examples, or exemplary language (e.g., "such as") provided herein is intended merely to better illuminate the invention.

The description herein of any aspect or embodiment of the invention using terms such as reference to an element or elements is intended to provide support for a similar aspect or embodiment of the invention that "consists of'," "consists essentially of" or "substantially comprises" that particular element or elements, unless otherwise stated or clearly contradicted by context (e. g. , a composition described as comprising a particular element should be understood as also describing a composition consisting of that element, unless otherwise stated or clearly contradicted by context).

### EXAMPLES

### Example 1: In vivo efficacy of Compound A in gastric PDX models relative to FGFR2 expression and/or amplification

11 PDX models of gastric cancer were selected considering their GCN and level of expression of FGFR2 as well as FGFR2 fusions, so as to constitute a balanced set of models with FGFR2 expression levels as well distributed as possible.

For each tumor model, the effects of Compound A on tumor growth (treatment efficacy), FGFR copy number and FGFR mRNA levels were assessed (or re-assessed). Tumor fragments from seed mice inoculated with selected PDX tumors were harvested and used for inoculation of female Balb/c nude mice. Each mouse was inoculated subcutaneously at the right flank with one tumor fragment (2-3 mm in diameter) for tumor development. Treatments were started when mean tumor size reached approximately 200-250mm³. Compound A (60-80 mg/kg) formulated as a suspension in 1% Kollidon VA64 in deionized water or vehicle alone (i.e., 1% Kollidon VA64 in deionized water) were administered orally by gavage to the tumor-bearing mice daily for 14 consecutive days. For each PDX model, 3 mice were used in the treatment group and 3 mice were used as control animals (vehicle only).

Tumor volume was measured twice weekly in two dimensions using a caliper, and the volume was expressed in mm³ using the formula: V = 0.5 a x *b*² where a and *b* are the long and short diameters of the tumor, respectively. Body weight was also recorded twice weekly. Treatment efficacy was expressed as ΔT/ΔC, whereby ΔT reports the relative change in tumor volume of drug-treated animals and ΔC the relative change in tumor volume of not-drug-treated animals between the last day of treatment and the beginning of treatment (median volume differences). Results obtained for the different models are shown in Table 6.

FGFR copy number was estimated by FISH. Tumor tissue obtained from tumor-bearing animals was fixed in buffered formalin and embedded in paraffin blocks (FFPE). Three to four µm tissue sections were mounted on silanized slides, deparaffinated, protease-treated, washed, DNA denatured and then hybridized to an FGFR probe as described by Schildhaus, H.U. et al. (2012) Mol. Pathol. 25: 1473-80. Subsequent to hybridization, tumor tissue was scanned for amplification hotspots using a fluorescent microscope as also described in Schildhaus et al. (2012). FGFR probes used were FGFR1/CEN 8 Dual Color Probe, FGFR2/CEN 10 Dual Color Probe and FGFR3/4p11 Dual Color Probe (all from Zytovision GmbH, Bremerhaven, Germany). Gene copy number gain (GCN) was defined as either amplification (FISH FGFR probe-centromere probe ratio ≥2.2) or polysomy, defined as FGFR probe-centromere probe ratio <2.2 but each one of FGFR and centromeres probes >2.

mRNA levels were determined by NanoString technology as described in the Methodology section above.

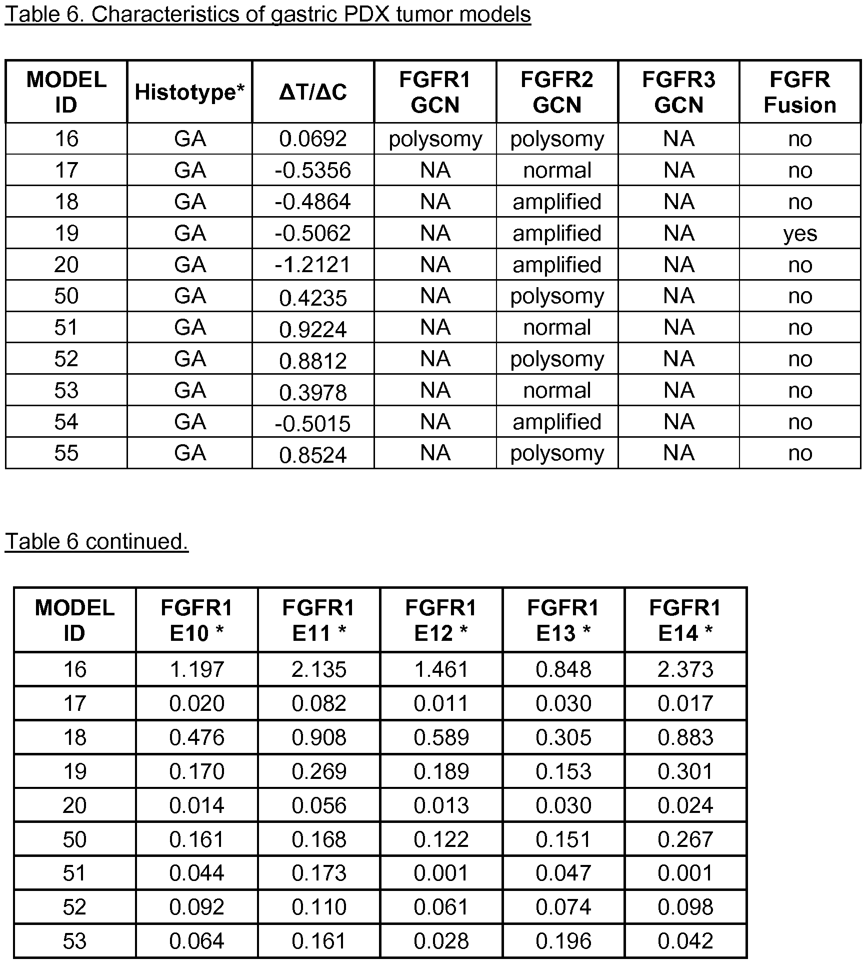

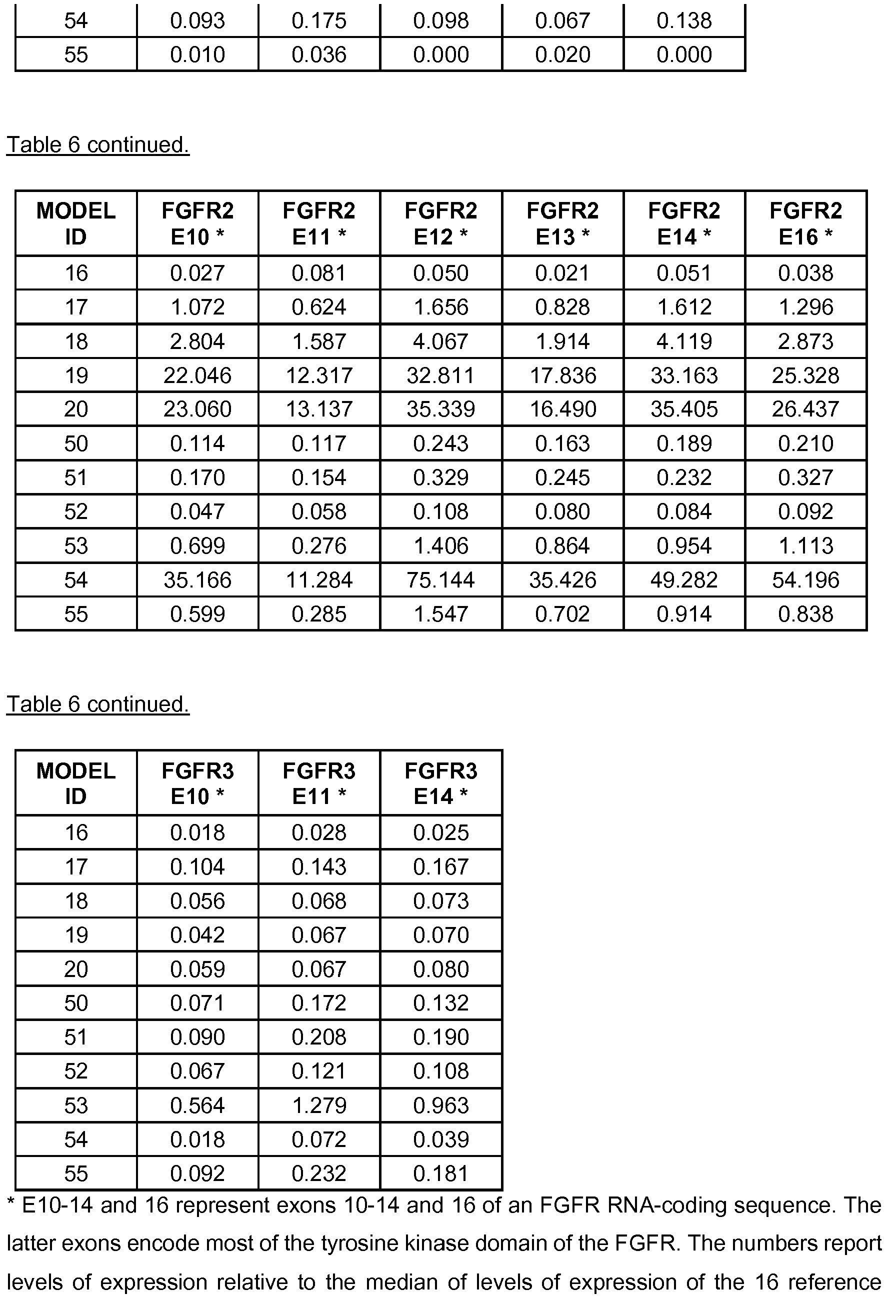

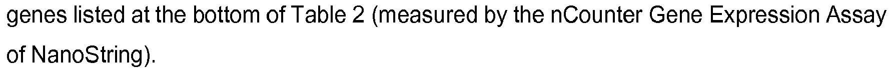

The relationship between treatment efficacy with Compound A (ΔT/ΔC) and FGFR2 mRNA expression (measured as expression level of one of the above-mentioned exons 10-14 and 16) are shown in Figure 10(A), wherein the models showing an FGFR2 amplification are also indicated (FISH ratio > 2.2, empty circles). It appears that an increased level of FGFR2 is associated with better anti-tumoral efficacy (Spearman correlation p-value: 0.0150174).

Figure 10(B) shows another representation of the same results clustered into two categories, namely responding models (ΔT/ΔC<0) and non-responding models (ΔT/ΔC>0) vs. FGFR2 mRNA expression levels, while also indicating FGFR2 amplification (FISH ratio > 2.2, empty circles).

It clearly appears that responding models and non-responding models displayed different levels of FGFR2 expression, and that selecting models (i.e. patient tumors) on the basis of FGFR2 amplification only (empty circles) does not allow to select all responding models. Indeed, one model displaying no amplification but high level of FGFR2 expression responded to Compound A.

### Example 2: In vivo efficacy of Compound A in esophageal squamous-cell carcinoma (ESCC) PDX models relative to FGFR1 expression and/or amplification

13 PDX models of ESCC were selected considering their GCN and level of expression of FGFR1, so as to constitute a balanced set of models with FGFR1 expression levels as well distributed as possible. In this particular ESCC indication, no FGFR1 fusions have been reported yet.

For each tumor model, the effects of Compound A on tumor growth (treatment efficacy), FGFR copy number and FGFR mRNA levels were assessed (or re-assessed). Tumor fragments from seed mice inoculated with selected PDX tumors were harvested and used for inoculation of female Balb/c nude mice. Each mouse was inoculated subcutaneously at the right flank with one tumor fragment (2-3 mm in diameter) for tumor development. Treatments were started when mean tumor size reached approximately 200-250mm³. Compound A (60-80 mg/kg) formulated as a suspension in 1% Kollidon VA64 in deionized water or vehicle alone (i.e., 1% Kollidon VA64 in deionized water) were administered orally by gavage to the tumor-bearing mice daily for 14 consecutive days. For each PDX model, 3 mice were used in the treatment group and 3 mice were used as control animals (vehicle only).

Tumor volume was measured twice weekly in two dimensions using a caliper, and the volume was expressed in mm³ using the formula: V = 0.5 a x *b*² where a and b are the long and short diameters of the tumor, respectively. Body weight was also recorded twice weekly. Treatment efficacy was expressed as ΔT/ΔC, whereby ΔT reports the relative change in tumor volume of drug-treated animals and ΔC the relative change in tumor volume of not-drug-treated animals between the last day of treatment and the beginning of treatment (median volume differences). Results obtained for the different models are shown in Table 7.

FGFR copy number was estimated by FISH. Tumor tissue obtained from tumor-bearing animals was fixed in buffered formalin and embedded in paraffin blocks (FFPE). Three to four µm tissue sections were mounted on silanized slides, deparaffinated, protease-treated, washed, DNA denatured and then hybridized to an FGFR probe as described by Schildhaus, H.U. et al. (2012) Mol. Pathol. 25: 1473-80. Subsequent to hybridization, tumor tissue was scanned for amplification hotspots using a fluorescent microscope as also described in Schildhaus et al. (2012). FGFR probes used were FGFR1/CEN 8 Dual Color Probe, FGFR2/CEN 10 Dual Color Probe and FGFR3/4p11 Dual Color Probe (all from Zytovision GmbH, Bremerhaven, Germany). Gene copy number gain (GCN) was defined as either amplification (FISH FGFR probe-centromere probe ratio ≥2.2) or polysomy, defined as FGFR probe-centromere probe ratio <2.2 but each one of FGFR and centromeres probes >2.

mRNA levels were determined by NanoString technology as described in the Methodology section above.

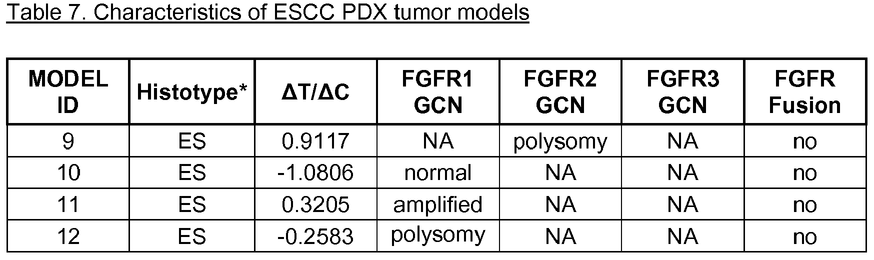

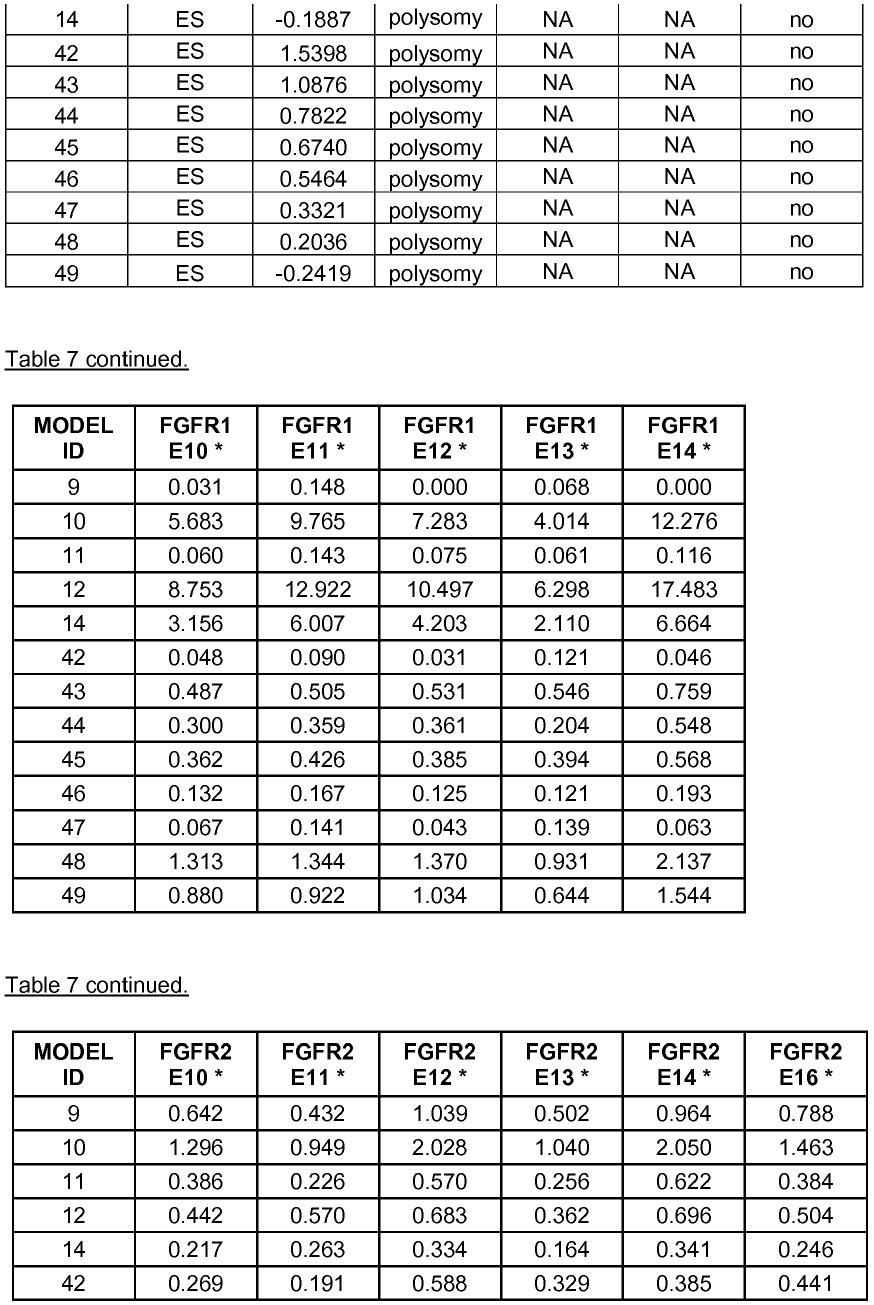

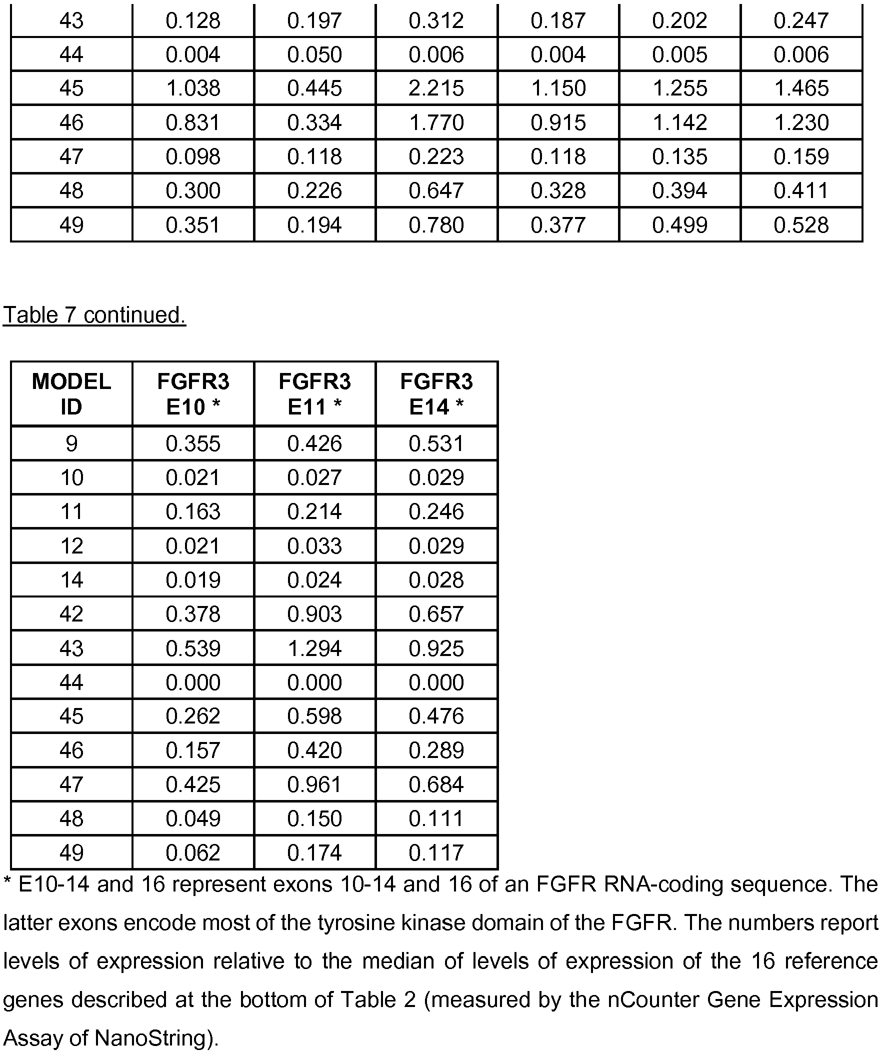

The relationship between treatment efficacy with Compound A (ΔT/ΔC) and FGFR1 mRNA expression (measured as expression level of one of the above-mentioned exons 10-14) are shown in Figure 11(A), wherein the models showing an FGFR1 amplification are also indicated (FISH ratio > 2.2, empty circles). It appears that an increased level of FGFR1 is associated with better anti-tumoral efficacy (Spearman correlation p-value: 0.0043872).

Figure 11(B) shows another representation of the same results clustered into two categories, namely responding models (ΔT/ΔC<0) and non-responding models (ΔT/ΔC>0) vs. FGFR1 mRNA expression levels, while also indicating FGFR1 amplification (FISH ratio > 2.2, empty circles).

It clearly appears that responding models and non-responding models displayed different levels of FGFR1 expression, and that for this particular ESCC indication, genetic structural alteration such as amplification (empty circles) does not allow to select models that respond to Compound A.

## Claims

1. A method for identifying a cancer likely to respond to Compound A, consisting of 5-amino-1-(2-methyl-1H-benzimidazol-5-yl)-1H-pyrazol-4-yl]-(1H-indol-2-yl)-methanone, comprising
(a) determining the levels of expression of FGFR1, FGFR2 and FGFR3 in a tumor or liquid biopsy from a subject suffering from a cancer, and
(b) if the determined level of expression of at least one of FGFR1, FGFR2 and FGFR3 exceeds a pre-established threshold level, determining that the subject has a cancer likely to respond to an effective amount of Compound A,
wherein levels of expression of FGFR1, FGFR2 and FGFR3 are measured at the messenger RNA level, and wherein the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

2. The method of claim 1, wherein the pre-established threshold level of at least one FGFR corresponds to:
(a) any level from the 60% to the 73% cutoff level of expression for the at least one FGFR, corresponding to expression levels relative to the median of levels of mRNA expression of a set of 16 reference genes measured by the nCounter Gene Expression Assay from 0.301 to 0.641 for FGFR1, from 0.669 to 1.094 for FGFR2, and from 0.289 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 65% to the 73% cutoff level of expression for the at least one FGFR, corresponding to expression levels relative to the median of levels of mRNA expression of a set of 16 reference genes measured by the nCounter Gene Expression Assay from 0.484 to 0.641 for FGFR1, from 0.884 to 1.094 for FGFR2, and from 0.490 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 73% cutoff level of expression for the at least one FGFR, corresponding to expression levels relative to the median of levels of mRNA expression of a set of 16 reference genes measured by the nCounter Gene Expression Assay from 0.558 to 0.641 for FGFR1, from 0.984 to 1.094 for FGFR2, and from 0.671 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

3. The method of claim 1, wherein the cancer is gastric cancer and the pre-established threshold level of expression of FGFR2 corresponds to:
(a) any level from the 44% to the 83% cutoff level of expression for FGFR2, corresponding to expression levels relative to the median of levels of mRNA expression of a set of 16 reference genes measured by the nCounter Gene Expression Assay from 0.257 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 83% cutoff level of expression for FGFR2, corresponding to expression levels relative to the median of levels of mRNA expression of a set of 16 reference genes measured by the nCounter Gene Expression Assay from 0.669 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 83% cutoff level of expression for FGFR2, corresponding to expression levels relative to the median of levels of mRNA expression of a set of 16 reference genes measured by the nCounter Gene Expression Assay from 0.984 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 83% cutoff level of expression for FGFR2, corresponding to expression levels relative to the median of levels of mRNA expression of a set of 16 reference genes measured by the nCounter Gene Expression Assay from 1.460 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

4. The method of claim 1, wherein the cancer is esophageal squamous-cell carcinoma and the pre-established threshold level of expression of FGFR1 corresponds to:
(a) any level from the 44% to the 84% cutoff level of expression for FGFR1, corresponding to expression levels relative to the median of levels of mRNA expression of a set of 16 reference genes measured by the nCounter Gene Expression Assay from 0.104 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 84% cutoff level of expression for FGFR1, corresponding to expression levels relative to the median of levels of mRNA expression of a set of 16 reference genes measured by the nCounter Gene Expression Assay from 0.301 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 84% cutoff level of expression for FGFR1, corresponding to expression levels relative to the median of levels of mRNA expression of a set of 16 reference genes measured by the nCounter Gene Expression Assay from 0.558 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 84% cutoff level of expression for FGFR1, corresponding to expression levels relative to the median of levels of mRNA expression of a set of 16 reference genes measured by the nCounter Gene Expression Assay from 0.759 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

5. A method of selecting a subject suffering from a cancer likely to respond to a therapeutic regimen that comprises a pharmaceutical composition comprising an effective amount of Compound A, the method comprising:
(a) determining the levels of expression of FGFR1, FGFR2 and FGFR3 in a tumor or liquid biopsy from the subject suffering from a cancer, and
(b) if the determined level of expression of at least one of FGFR1, FGFR2 and FGFR3 exceeds a pre-established threshold level, considering the subject eligible for the therapeutic regimen,
wherein levels of expression of FGFR1, FGFR2 and FGFR3 are measured at the messenger RNA level and wherein the pre-established threshold level of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

6. The method of claim 5, wherein the pre-established threshold level of the at least one FGFR corresponds to:
(a) any level from the 60% to the 73% cutoff level of expression for the at least one FGFR, corresponding to expression levels relative to the median of levels of mRNA expression of a set of 16 reference genes measured by the nCounter Gene Expression Assay from 0.301 to 0.641 for FGFR1, from 0.669 to 1.094 for FGFR2, and from 0.289 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 65% to the 73% cutoff level of expression for the at least one FGFR, corresponding to expression levels relative to the median of levels of mRNA expression of a set of 16 reference genes measured by the nCounter Gene Expression Assay from 0.484 to 0.641 for FGFR1, from 0.884 to 1.094 for FGFR2, and from 0.490 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 73% cutoff level of expression for the at least one FGFR, corresponding to expression levels relative to the median of levels of mRNA expression of a set of 16 reference genes measured by the nCounter Gene Expression Assay from 0.558 to 0.641 for FGFR1, from 0.984 to 1.094 for FGFR2, and from 0.671 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

7. The method of claim 5, wherein the cancer is gastric cancer and the pre-established threshold level of expression of FGFR2 corresponds to:
(a) any level from the 44% to the 83% cutoff level of expression for FGFR2, corresponding to expression levels relative to the median of levels of mRNA expression of a set of 16 reference genes measured by the nCounter Gene Expression Assay from 0.257 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 83% cutoff level of expression for FGFR2, corresponding to expression levels relative to the median of levels of mRNA expression of a set of 16 reference genes measured by the nCounter Gene Expression Assay from 0.669 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 83% cutoff level of expression for FGFR2, corresponding to expression levels relative to the median of levels of mRNA expression of a set of 16 reference genes measured by the nCounter Gene Expression Assay from 0.984 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 83% cutoff level of expression for FGFR2, corresponding to expression levels relative to the median of levels of mRNA expression of a set of 16 reference genes measured by the nCounter Gene Expression Assay from 1.460 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

8. The method of claim 5, wherein the cancer is esophageal squamous-cell carcinoma and the pre-established threshold level of expression of FGFR1 corresponds to:
(a) any level from the 44% to the 84% cutoff level of expression for FGFR1, corresponding to expression levels relative to the median of levels of mRNA expression of a set of 16 reference genes measured by the nCounter Gene Expression Assay from 0.104 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 84% cutoff level of expression for FGFR1, corresponding to expression levels relative to the median of levels of mRNA expression of a set of 16 reference genes measured by the nCounter Gene Expression Assay from 0.301 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 84% cutoff level of expression for FGFR1, corresponding to expression levels relative to the median of levels of mRNA expression of a set of 16 reference genes measured by the nCounter Gene Expression Assay from 0.558 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 84% cutoff level of expression for FGFR1, corresponding to expression levels relative to the median of levels of mRNA expression of a set of 16 reference genes measured by the nCounter Gene Expression Assay from 0.759 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

9. Compound A for use in the treatment of cancer in a subject, wherein the level of expression of at least one of FGFR1, FGFR2 and FGFR3 as determined in a tumor or liquid biopsy from the subject exceeds a pre-established threshold level,
wherein levels of expression of FGFR1, FGFR2 and FGFR3 are measured at the messenger RNA level and wherein the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

10. Compound A for use according to claim 9, wherein the pre-established threshold level of expression of at least one FGFR corresponds to:
(a) any level from the 60% to the 73% cutoff level of expression for the at least one FGFR, corresponding to expression levels relative to the median of levels of mRNA expression of a set of 16 reference genes measured by the nCounter Gene Expression Assay from 0.301 to 0.641 for FGFR1, from 0.669 to 1.094 for FGFR2, and from 0.289 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 65% to the 73% cutoff level of expression for the at least one FGFR, corresponding to expression levels relative to the median of levels of mRNA expression of a set of 16 reference genes measured by the nCounter Gene Expression Assay from 0.484 to 0.641 for FGFR1, from 0.884 to 1.094 for FGFR2, and from 0.490 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 73% cutoff level of expression for the at least one FGFR, corresponding to expression levels relative to the median of levels of mRNA expression of a set of 16 reference genes measured by the nCounter Gene Expression Assay from 0.558 to 0.641 for FGFR1, from 0.984 to 1.094 for FGFR2, and from 0.671 to 0.815 for FGFR3, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

11. The compound A for use according to claim 9, wherein the cancer is gastric cancer and the pre-established threshold level of expression of FGFR2 corresponds to
(a) any level from the 44% to the 83% cutoff level of expression for FGFR2, corresponding to expression levels relative to the median of levels of mRNA expression of a set of 16 reference genes measured by the nCounter Gene Expression Assay from 0.257 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 83% cutoff level of expression for FGFR2, corresponding to expression levels relative to the median of levels of mRNA expression of a set of 16 reference genes measured by the nCounter Gene Expression Assay from 0.669 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 83% cutoff level of expression for FGFR2, corresponding to expression levels relative to the median of levels of mRNA expression of a set of 16 reference genes measured by the nCounter Gene Expression Assay from 0.984 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 83% cutoff level of expression for FGFR2, corresponding to expression levels relative to the median of levels of mRNA expression of a set of 16 reference genes measured by the nCounter Gene Expression Assay from 1.460 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

12. The compound A for use according to claim 9, wherein the cancer is esophageal squamous-cell carcinoma and the pre-established threshold level of expression of FGFR1 corresponds to
(a) any level from the 44% to the 84% cutoff level of expression for FGFR1, corresponding to expression levels relative to the median of levels of mRNA expression of a set of 16 reference genes measured by the nCounter Gene Expression Assay from 0.104 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(b) any level from the 60% to the 84% cutoff level of expression for FGFR1, corresponding to expression levels relative to the median of levels of mRNA expression of a set of 16 reference genes measured by the nCounter Gene Expression Assay from 0.301 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(c) any level from the 70% to the 84% cutoff level of expression for FGFR1, corresponding to expression levels relative to the median of levels of mRNA expression of a set of 16 reference genes measured by the nCounter Gene Expression Assay from 0.558 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6, or
(d) any level from the 80% to the 84% cutoff level of expression for FGFR1, corresponding to expression levels relative to the median of levels of mRNA expression of a set of 16 reference genes measured by the nCounter Gene Expression Assay from 0.759 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

13. A method of selecting a subject suffering from a cancer for treatment with Compound A, the method comprising determining the levels of expression of FGFR1, FGFR2 and FGFR3 in a tumor or liquid biopsy from the subject and if the determined level of expression of at least one of FGFR1, FGFR2 and FGFR3 exceeds a pre-established threshold level, considering the subject eligible for treatment, wherein levels of expression of FGFR1, FGFR2 and FGFR3 are measured at the messenger RNA level or at the protein level, and wherein the pre-established threshold level of expression of at least one FGFR corresponds to any level from the 44% to the 73% cutoff level of expression for the at least one FGFR, corresponding to expression levels relative to the median of levels of mRNA expression of a set of 16 reference genes measured by the nCounter Gene Expression Assay from 0.104 to 0.641 for FGFR1, from 0.257 to 1.094 for FGFR2, and from 0.128 to 0.815 for FGFR3, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

14. The method of claim 13, wherein the cancer is a gastric cancer and the subject is considered eligible for treatment if the level of expression of FGFR2 exceeds a pre-established threshold level, wherein the pre-established threshold level of expression of FGFR2 corresponds to any level from the 44% to the 83% cutoff level of expression for FGFR2, corresponding to expression levels relative to the median of levels of mRNA expression of a set of 16 reference genes measured by the nCounter Gene Expression Assay from 0.257 to 1.610 for FGFR2, the 16 reference genes being ACTB, ALAS1, CLTC, MRPL19, RPL19, RPLP0, SF3A1, TBP, TUBB, c1orf43 exon 1, c1orf43 exon 2, CHMP2A exon 3, EMC7 exon 5, EMC7 exon 3, GPI exon 4 and GPI exon 6.

15. The method of claim 13, wherein the cancer is an esophageal squamous-cell carcinoma and the subject is considered eligible for treatment if the level of expression of FGFR1 exceeds a pre-established threshold level, wherein the pre-established threshold level of expression of FGFR1 corresponds to any level from the 44% to the 84% cutoff level of expression for FGFR1, corresponding to expression levels relative to the median of levels of mRNA expression of a set of 16 reference genes measured by the nCounter Gene Expression Assay from 0.104 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

16. A method of selecting a subject suffering from a gastric cancer for treatment with Compound A, the method comprising determining the level of expression FGFR2 in a tumor or liquid biopsy from the subject and if the determined level of expression of FGFR2 exceeds a pre-established threshold level, considering the subject eligible for treatment, wherein the pre-established threshold level of expression of FGFR2 corresponds to any level from the 44% to the 83% cutoff level of expression for FGFR2, corresponding to expression levels relative to the median of levels of mRNA expression of a set of 16 reference genes measured by the nCounter Gene Expression Assay from 0.257 to 1.610 for FGFR2, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

17. A method of selecting a subject suffering from an esophageal squamous-cell carcinoma for treatment with Compound A, the method comprising determining the level of expression FGFR1 in a tumor or liquid biopsy from the subject and if the determined level of expression of FGFR1 exceeds a pre-established threshold level, considering the subject eligible for treatment, wherein the pre-established threshold level of expression of FGFR1 corresponds to any level from the 44% to the 84% cutoff level of expression for FGFR1, corresponding to expression levels relative to the median of levels of mRNA expression of a set of 16 reference genes measured by the nCounter Gene Expression Assay from 0.104 to 1.362 for FGFR1, the 16 reference genes being ACTB, ALAS1, CLTC, MRPL19, RPL19, RPLPO, SF3A1, TBP, TUBB, c1orf43 exon 1, c1orf43 exon 2, CHMP2A exon 3, EMC7 exon 5, EMC7 exon 3, GPI exon 4 and GPI exon 6.

## Patentansprüche

1. Verfahren zu Identifizieren eines Krebses, der voraussichtlich auf Verbindung A bestehend aus 5-Amino-1-(2-methyl-1H-benzimidazol-5-yl)-1H-pyrazol-4-yl]-(1H-indol-2-yl)-methanon anspricht, umfassend:
(a) Bestimmen der Expressionsniveaus von FGFR1, FGFR2 und FGFR3 in einer Tumor- oder Flüssigkeitsbiopsie von einem Individuum, das an einem Krebs leidet, und
(b) Bestimmen, wenn das bestimmte Expressionsniveau von mindestens einem von FGFR1, FGFR2 und FGFR3 einen vorab festgelegten Schwellenwert überschreitet, dass das Individuum einen Krebs hat, der voraussichtlich auf eine wirksame Menge von Verbindung A anspricht,
wobei Expressionsniveaus von FGFR1, FGFR2 und FGFR3 auf der Messenger-RNA-Ebene gemessen werden und wobei der vorab festgelegte Schwellenwert der Expression von mindestens einem FGFR einem beliebigen Wert des Expressionsgrenzwerts zwischen dem 44. und dem 73. Perzentil für den mindestens einen FGFR entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 0,641 für FGFR1, von 0,257 bis 1,094 für FGFR2 und von 0,128 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

2. Verfahren nach Anspruch 1, wobei der vorab festgelegte Schwellenwert von mindestens einem FGFR folgendem entspricht:
(a) einem beliebigen Wert des Expressionsgrenzwerts zwischen dem 60. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,301 bis 0,641 für FGFR1, von 0,669 bis 1,094 für FGFR2 und von 0,289 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 65. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,484 bis 0,641 für FGFR1, von 0,884 bis 1,094 für FGFR2 und von 0,490 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,558 bis 0,641 für FGFR1, von 0,984 bis 1,094 für FGFR2 und von 0,671 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

3. Verfahren nach Anspruch 1, wobei es sich bei dem Krebs um einen Magenkrebs handelt und der vorab festgelegte Schwellenwert der Expression von FGFR2 folgendem entspricht:
(a) einem beliebigen Wert des Expressionsgrenzwerts zwischen dem 44. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,257 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,669 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,984 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 1,460 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

4. Verfahren nach Anspruch 1, wobei es sich bei dem Krebs um ein ösophageales Plattenepithelzellkarzinom handelt und der vorab festgelegte Schwellenwert der Expression von FGFR1 folgendem entspricht:
(a) einem beliebigen Wert des Expressionsgrenzwerts zwischen dem 44. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,104 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,301 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,558 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,759 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

5. Verfahren zum Auswählen eines Individuums, das an einem Krebs leidet, der voraussichtlich auf einen Therapieplan anspricht, welcher eine pharmazeutische Zusammensetzung umfassend eine wirksame Menge von Verbindung A umfasst, wobei das Verfahren folgendes umfasst:
(a) Bestimmen der Expressionsniveaus von FGFR1, FGFR2 und FGFR3 in einer Tumor- oder Flüssigkeitsbiopsie von dem Individuum, das an einem Krebs leidet, und
(b) Geeignet-halten des Individuums, wenn das bestimmte Expressionsniveau von mindestens einem von FGFR1, FGFR2 und FGFR3 einen vorab festgelegten Schwellenwert überschreitet, für den Therapieplan,
wobei Expressionsniveaus von FGFR1, FGFR2 und FGFR3 auf der Messenger-RNA-Ebene gemessen werden und wobei der vorab festgelegte Schwellenwert der Expression von mindestens einem FGFR einem beliebigen Wert des Expressionsgrenzwerts zwischen dem 44. und dem 73. Perzentil für den mindestens einen FGFR entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 0,641 für FGFR1, von 0,257 bis 1,094 für FGFR2 und von 0,128 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

6. Verfahren nach Anspruch 5, wobei der vorab festgelegte Schwellenwert des mindestens einen FGFR folgendem entspricht:
(a) einem beliebigen Wert des Expressionsgrenzwerts zwischen dem 60. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,301 bis 0,641 für FGFR1, von 0,669 bis 1,094 für FGFR2 und von 0,289 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 65. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,484 bis 0,641 für FGFR1, von 0,884 bis 1,094 für FGFR2 und von 0,490 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,558 bis 0,641 für FGFR1, von 0,984 bis 1,094 für FGFR2 und von 0,671 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

7. Verfahren nach Anspruch 5, wobei es sich bei dem Krebs um einen Magenkrebs handelt und der vorab festgelegte Schwellenwert der Expression von FGFR2 folgendem entspricht:
(a) einem beliebigen Wert des Expressionsgrenzwerts zwischen dem 44. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,257 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,669 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,984 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 1,460 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

8. Verfahren nach Anspruch 5, wobei es sich bei dem Krebs um ein ösophageales Plattenepithelzellkarzinom handelt und der vorab festgelegte Schwellenwert der Expression von FGFR1 folgendem entspricht:
(a) einem beliebigen Wert des Expressionsgrenzwerts zwischen dem 44. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,104 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,301 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,558 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,759 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

9. Verbindung A zur Verwendung bei der Behandlung von Krebs in einem Individuum, wobei das Expressionsniveau von mindestens einem von FGFR1, FGFR2 und FGFR3, wie in einer Tumor- oder Flüssigkeitsbiopsie von dem Individuum bestimmt, einen vorab festgelegten Schwellenwert überschreitet,
wobei Expressionsniveaus von FGFR1, FGFR2 und FGFR3 auf der Messenger-RNA-Ebene gemessen werden und wobei der vorab festgelegte Schwellenwert der Expression von mindestens einem FGFR einem beliebigen Wert des Expressionsgrenzwerts zwischen dem 44. und dem 73. Perzentil für den mindestens einen FGFR entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 0,641 für FGFR1, von 0,257 bis 1,094 für FGFR2 und von 0,128 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

10. Verbindung A zur Verwendung nach Anspruch 9, wobei der vorab festgelegte Schwellenwert der Expression von mindestens einem FGFR folgendem entspricht:
(a) einem beliebigen Wert des Expressionsgrenzwerts zwischen dem 60. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,301 bis 0,641 für FGFR1, von 0,669 bis 1,094 für FGFR2 und von 0,289 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 65. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,484 bis 0,641 für FGFR1, von 0,884 bis 1,094 für FGFR2 und von 0,490 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 73. Perzentil für den mindestens einen FGFR, entsprechend Expressionsniveaus relativ zu dem Median von Expressionsniveaus von mRNA eines Satzes von 16 Referenzgenen, gemessen mit dem nCounter-Genexpressionstest, von 0,558 bis 0,641 für FGFR1, von 0,984 bis 1,094 für FGFR2 und von 0,671 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

11. Verbindung A zur Verwendung nach Anspruch 9, wobei es sich bei dem Krebs um einen Magenkrebs handelt und der vorab festgelegte Schwellenwert der Expression von FGFR2 folgendem entspricht:
(a) einem beliebigen Wert des Expressionsgrenzwerts zwischen dem 44. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,257 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,669 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 0,984 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 83. Perzentil für FGFR2, entsprechend Expressionsniveaus relativ zu dem Median von Expressionsniveaus von mRNA eines Satzes von 16 Referenzgenen, gemessen mit dem nCounter-Genexpressionstest, von 1,460 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

12. Verbindung A zur Verwendung nach Anspruch 9, wobei es sich bei dem Krebs um ein ösophageales Plattenepithelzellkarzinom handelt und der vorab festgelegte Schwellenwert der Expression von FGFR1 folgendem entspricht:
(a) einem beliebigen Wert des Expressionsgrenzwerts zwischen dem 44. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,104 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(b) einem beliebigen Wert des Expressionsgrenzwerts zwischen dem 60. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,301 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(c) einem beliebigen Wert des Expressionsgrenzwerts zwischen dem 70. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,558 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt, oder
(d) einem beliebigen Wert des Expressionsgrenzwerts zwischen dem 80. und dem 84. Perzentil für FGFR1, entsprechend Expressionsniveaus relativ zu dem Median von Expressionsniveaus von mRNA eines Satzes von 16 Referenzgenen, gemessen mit dem nCounter-Genexpressionstest, von 0,759 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

13. Verfahren zum Auswählen eines Individuums, das an einem Krebs leidet, für die Behandlung mit Verbindung A, wobei das Verfahren das Bestimmen der Expressionsniveaus von FGFR1, FGFR2 und FGFR3 in einer Tumor- oder Flüssigkeitsbiopsie von dem Individuum und, wenn das bestimmte Expressionsniveau von mindestens einem von FGFR1, FGFR2 und FGFR3 einen vorab festgelegten Schwellenwert überschreitet, Geeignet-halten des Individuums für die Behandlung umfasst, wobei Expressionsniveaus von FGFR1, FGFR2 und FGFR3 auf der Messenger-RNA-Ebene oder auf der Proteinebene gemessen werden und wobei der vorab festgelegte Schwellenwert der Expression von mindestens einem FGFR einem beliebigen Wert des Expressionsgrenzwerts zwischen dem 44. und dem 73. Perzentil für den mindestens einen FGFR entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 0,641 für FGFR1, von 0,257 bis 1,094 für FGFR2 und von 0,128 bis 0,815 für FGFR3, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

14. Verfahren nach Anspruch 13, wobei es sich bei dem Krebs um einen Magenkrebs handelt und das Individuum als für die Behandlung geeignet gehalten wird, wenn das Expressionsniveau von FGFR2 einen vorab festgelegten Schwellenwert überschreitet, wobei der vorab festgelegte Schwellenwert der Expression von FGFR2 einem beliebigen Wert des Expressionsgrenzwerts zwischen dem 44. und dem 83. Perzentil für FGFR2 entspricht, entsprechend Expressionsniveaus relativ zu dem Median von Expressionsniveaus von mRNA eines Satzes von 16 Referenzgenen, gemessen mit dem nCounter-Genexpressionstest, von 0,257 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

15. Verfahren nach Anspruch 13, wobei es sich bei dem Krebs um ein ösophageales Plattenepithelzellkarzinom handelt und das Individuum als für die Behandlung geeignet gehalten wird, wenn das Expressionsniveau von FGFR1 einen vorab festgelegten Schwellenwert überschreitet, wobei der vorab festgelegte Schwellenwert der Expression von FGFR1 einem beliebigen Wert des Expressionsgrenzwerts zwischen dem 44. und dem 84. Perzentil für FGFR1 entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 1,362 für FGFR1, wobei es sich bei den 16 Referenzgenen um ACTB, ALAS1, CLTC, MRPL19, RPL19, RPLP0, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

16. Verfahren zum Auswählen eines Individuums, das an einem Magenkrebs leidet, für die Behandlung mit Verbindung A, wobei das Verfahren das Bestimmen des Expressionsniveaus von FGFR2 in einer Tumor- oder Flüssigkeitsbiopsie von dem Individuum und, wenn das bestimmte Expressionsniveau von FGFR2 einen vorab festgelegten Schwellenwert überschreitet, Geeignet-halten des Individuums für die Behandlung umfasst, wobei der vorab festgelegte Schwellenwert der Expression von FGFR2 einem beliebigen Wert des Expressionsgrenzwerts zwischen dem 44. und dem 83. Perzentil für FGFR2 entspricht, entsprechend Expressionsniveaus relativ zu dem Median von Expressionsniveaus von mRNA eines Satzes von 16 Referenzgenen, gemessen mit dem nCounter-Genexpressionstest, von 0,257 bis 1,610 für FGFR2, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

17. Verfahren zum Auswählen eines Individuums, das an einem ösophagealen Plattenepithelzellkarzinom leidet, für die Behandlung mit Verbindung A, wobei das Verfahren das Bestimmen des Expressionsniveaus von FGFR1 in einer Tumor- oder Flüssigkeitsbiopsie von dem Individuum und, wenn das bestimmte Expressionsniveau von FGFR1 einen vorab festgelegten Schwellenwert überschreitet, Geeignet-halten des Individuums für die Behandlung umfasst, wobei der vorab festgelegte Schwellenwert der Expression von FGFR1 einem beliebigen Wert des Expressionsgrenzwerts zwischen dem 44. und dem 84. Perzentil für FGFR1 entspricht, entsprechend Expressionsniveaus relativ zu dem Median von Expressionsniveaus von mRNA eines Satzes von 16 Referenzgenen, gemessen mit dem nCounter-Genexpressionstest, von 0,104 bis 1,362 für FGFR1, wobei es sich bei den 16 Referenzgenen um ACTB, ALAS1, CLTC, MRPL19, RPL19, RPLPO, SF3A1, TBP, TUBB, c1orf43-Exon 1, c1orf43-Exon 2, CHMP2A-Exon 3, EMC7-Exon 5, EMC7-Exon 3, GPI-Exon 4 und GPI-Exon 6 handelt.

## Revendications

1. Procédé d'identification d'un cancer susceptible de réagir au composé A, consistant en la 5-amino-1-(2-méthyl-1H-benzimidazol-5-yl)-1H-pyrazol-4-yl]-(1H-indol-2-yl)-méthanone, comprenant le fait
(a) de déterminer les niveaux d'expression de FGFR1, FGFR2 et FGFR3 dans une biopsie de tumeur ou liquide d'un sujet souffrant d'un cancer, et
(b) si le niveau d'expression déterminé d'au moins l'un de FGFR1, FGFR2 et FGFR3 dépasse un niveau seuil préétabli, de déterminer que le sujet a un cancer susceptible de réagir à une quantité efficace du composé A,
dans lequel les niveaux d'expression de FGFR1, FGFR2 et FGFR3 sont mesurés au niveau de l'ARN messager, et dans lequel le niveau seuil préétabli d'expression d'au moins un FGFR correspond à un niveau d'expression quelconque entre le 44^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,104 à 0,641 pour FGFR1, de 0,257 à 1,094 pour FGFR2 et de 0,128 à 0,815 pour FGFR3, les 16 gènes de référence étant ACTB, ALAS1, CLTC, MRPL19, RPL19, RPLP0, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

2. Le procédé de la revendication 1, dans lequel le niveau seuil préétabli d'au moins un FGFR correspond à :
(a) un niveau d'expression quelconque entre le 60^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,301 à 0,641 pour FGFR1, de 0,669 à 1,094 pour FGFR2 et de 0,289 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 65^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,484 à 0,641 pour FGFR1, de 0,884 à 1,094 pour FGFR2 et de 0,490 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,558 à 0,641 pour FGFR1, de 0,984 à 1,094 pour FGFR2 et de 0,671 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

3. Le procédé de la revendication 1, dans lequel le cancer est un cancer gastrique et le niveau seuil préétabli d'expression de FGFR2 correspond à :
(a) un niveau d'expression quelconque entre le 44^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,257 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,669 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,984 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 1,460 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

4. Le procédé de la revendication 1, dans lequel le cancer est un carcinome squameux de l'oesophage et le niveau seuil préétabli d'expression de FGFR1 correspond à :
(a) un niveau d'expression quelconque entre le 44^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,104 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,301 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,558 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,759 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

5. Procédé de sélection d'un sujet souffrant d'un cancer susceptible de réagir à un régime thérapeutique qui comprend une composition pharmaceutique comprenant une quantité efficace du composé A, le procédé comprenant le fait :
(a) de déterminer les niveaux d'expression de FGFR1, FGFR2 et FGFR3 dans une biopsie de tumeur ou liquide du sujet souffrant d'un cancer, et
(b) si le niveau d'expression déterminé d'au moins l'un de FGFR1, FGFR2 et FGFR3 dépasse un niveau seuil préétabli, de considérer le sujet comme éligible au régime thérapeutique,
dans lequel les niveaux d'expression de FGFR1, FGFR2 et FGFR3 sont mesurés au niveau de l'ARN messager et dans lequel le niveau seuil préétabli d'au moins un FGFR correspond à un niveau d'expression quelconque entre le 44^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,104 à 0,641 pour FGFR1, de 0,257 à 1,094 pour FGFR2 et de 0,128 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

6. Le procédé de la revendication 5, dans lequel le niveau seuil préétabli d'au moins un FGFR correspond à :
(a) un niveau d'expression quelconque entre le 60^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,301 à 0,641 pour FGFR1, de 0,669 à 1,094 pour FGFR2 et de 0,289 à 0,815 pour FGFR3, les 16 gènes de référence étant ACTB, ALAS1, CLTC, MRPL19, RPL19, RPLP0, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 65^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,484 à 0,641 pour FGFR1, de 0,884 à 1,094 pour FGFR2 et de 0,490 à 0,815 pour FGFR3, les 16 gènes de référence étant ACTB, ALAS1, CLTC, MRPL19, RPL19, RPLP0, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,558 à 0,641 pour FGFR1, de 0,984 à 1,094 pour FGFR2 et de 0,671 à 0,815 pour FGFR3, les 16 gènes de référence étant ACTB, ALAS1, CLTC, MRPL19, RPL19, RPLP0, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

7. Le procédé de la revendication 5, dans lequel le cancer est un cancer gastrique et le niveau seuil préétabli d'expression de FGFR2 correspond à :
(a) un niveau d'expression quelconque entre le 44^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,257 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,669 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,984 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 1,460 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

8. Le procédé de la revendication 5, dans lequel le cancer est un carcinome squameux de l'oesophage et le niveau seuil préétabli d'expression de FGFR1 correspond à :
(a) un niveau d'expression quelconque entre le 44^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,104 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,301 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,558 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,759 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

9. Composé A pour son utilisation dans le traitement du cancer chez un sujet, dans lequel le niveau d'expression d'au moins l'un de FGFR1, FGFR2 et FGFR3 tel que déterminé dans une biopsie de tumeur ou liquide du sujet dépasse un niveau seuil préétabli,
dans lequel les niveaux d'expression de FGFR1, FGFR2 et FGFR3 sont mesurés au niveau de l'ARN messager et dans lequel le niveau seuil préétabli d'expression d'au moins un FGFR correspond à un niveau d'expression quelconque entre le 44^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,104 à 0,641 pour FGFR1, de 0,257 à 1,094 pour FGFR2 et de 0,128 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

10. Le composé A pour son utilisation selon la revendication 9, dans lequel le niveau seuil préétabli d'expression d'au moins un FGFR correspond à :
(a) un niveau d'expression quelconque entre le 60^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,301 à 0,641 pour FGFR1, de 0,669 à 1,094 pour FGFR2 et de 0,289 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 65^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,484 à 0,641 pour FGFR1, de 0,884 à 1,094 pour FGFR2 et de 0,490 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,558 à 0,641 pour FGFR1, de 0,984 à 1,094 pour FGFR2 et de 0,671 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

11. Le composé A pour son utilisation selon la revendication 9, dans lequel le cancer est un cancer gastrique et le niveau seuil préétabli d'expression de FGFR2 correspond à
(a) un niveau d'expression quelconque entre le 44^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,257 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,669 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,984 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 1,460 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

12. Le composé A pour son utilisation selon la revendication 9, dans lequel le cancer est un carcinome squameux de l'oesophage et le niveau seuil préétabli d'expression de FGFR1 correspond à
(a) un niveau d'expression quelconque entre le 44^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,104 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(b) un niveau d'expression quelconque entre le 60^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,301 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(c) un niveau d'expression quelconque entre le 70^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,558 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI, ou
(d) un niveau d'expression quelconque entre le 80^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,759 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

13. Un procédé de sélection d'un sujet souffrant d'un cancer pour le traitement avec le composé A, le procédé comprenant le fait de déterminer les niveaux d'expression de FGFR1, FGFR2 et FGFR3 dans une biopsie de tumeur ou liquide du sujet et si le niveau d'expression déterminé d'au moins l'un de FGFR1, FGFR2 et FGFR3 dépasse un niveau seuil préétabli, de considérer le sujet comme éligible au traitement, dans lequel les niveaux d'expression de FGFR1, FGFR2 et FGFR3 sont mesurés au niveau de l'ARN messager ou au niveau protéine, et dans lequel le niveau seuil préétabli d'expression d'au moins un FGFR correspond à un niveau d'expression quelconque entre le 44^{e} et le 73^{e} percentile du niveau d'expression pour l'au moins un FGFR, correspondant aux niveaux d'expression par rapport à la médiane des niveaux d'expression d'ARNm d'un ensemble de 16 gènes de référence mesurés par le Dosage d'Expression Génétique nCounter allant de 0,104 à 0,641 pour FGFR1, de 0,257 à 1,094 pour FGFR2 et de 0,128 à 0,815 pour FGFR3, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

14. Le procédé de la revendication 13, dans lequel le cancer est un cancer gastrique et le sujet est considéré comme éligible au traitement si le niveau d'expression du FGFR2 dépasse un niveau seuil préétabli, dans lequel le niveau seuil préétabli d'expression de FGFR2 correspond à un niveau d'expression quelconque entre le 44^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,257 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

15. Le procédé de la revendication 13, dans lequel le cancer est un carcinome squameux de l'oesophage et le sujet est considéré comme éligible au traitement si le niveau d'expression de FGFR1 dépasse un niveau seuil préétabli, dans lequel le niveau seuil préétabli d'expression de FGFR1 correspond à un niveau d'expression quelconque entre le 44^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,104 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

16. Un procédé de sélection d'un sujet souffrant d'un cancer gastrique pour le traitement avec le composé A, le procédé comprenant le fait de déterminer le niveau d'expression de FGFR2 dans une biopsie de tumeur ou liquide du sujet et si le niveau d'expression déterminé de FGFR2 dépasse un niveau seuil préétabli, de considérer le sujet comme éligible au traitement, dans lequel le niveau seuil préétabli d'expression de FGFR2 correspond à un niveau d'expression quelconque entre le 44^{e} et le 83^{e} percentile du niveau d'expression pour FGFR2, correspondant aux niveaux d'expression par rapport à la médiane des niveaux d'expression d'ARNm d'un ensemble de 16 gènes de référence mesurés par le Dosage d'Expression Génétique nCounter allant de 0,257 à 1,610 pour FGFR2, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.

17. Un procédé de sélection d'un sujet souffrant d'un carcinome squameux de l'oesophage pour le traitement avec le composé A, le procédé comprenant le fait de déterminer le niveau d'expression de FGFR1 dans une biopsie de tumeur ou liquide du sujet et si le niveau d'expression déterminé de FGFR1 dépasse un niveau seuil préétabli, de considérer le sujet comme éligible au traitement, dans lequel le niveau seuil préétabli d'expression de FGFR1 correspond à un niveau d'expression quelconque entre le 44^{e} et le 84^{e} percentile du niveau d'expression pour FGFR1, correspondant aux niveaux d'expression par rapport à la médiane des niveaux d'expression d'ARNm d'un ensemble de 16 gènes de référence mesurés par le Dosage d'Expression Génétique nCounter allant de 0,104 à 1,362 pour FGFR1, les 16 gènes de référence étant ACTB, ALAS1, CLTC, MRPL19, RPL19, RPLPO, SF3A1, TBP, TUBB, l'exon 1 de c1orf43, l'exon 2 de c1orf43, l'exon 3 de CHMP2A, l'exon 5 d'EMC7, l'exon 3 d'EMC7, l'exon 4 de GPI et l'exon 6 de GPI.
